# EUROPEAN PATENT SPECIFICATION

(11) **EP 4 030 993 B1**
(45) Date of publication and mention of the grant of the patent: **15.04.2026**
(21) Application number: 20864655.4
(22) Date of filing: 16.09.2020
(51) Int. Cl.: A61B 5/107, A61B 5/11

(54) **IMPROVEMENTS IN PERSONALIZED HEALTHCARE FOR PATIENTS WITH MOVEMENT DISORDERS**
PERSONALISIERTE GESUNDHEITSPFLEGE FÜR PATIENTEN MIT BEWEGUNGSSTÖRUNGEN
AMÉLIORATIONS APPORTÉES À DES SOINS DE SANTÉ PERSONNALISÉS DESTINÉS À DES PATIENTS ATTEINTS DE TROUBLES DE LA MOTRICITÉ

(30) Priority: 17.09.2019 US 201962901461 P
(43) Date of publication of application: 27.07.2022
(73) Proprietor: F. Hoffmann-La Roche AG, 4070 Basel (CH)
(72) Inventor: LIPSMEIER, Florian, 4070 Basel (CH); LINDEMANN, Michael, 4051 Basel (CH); TAYLOR, Kirsten, 4070 Basel (CH); THOMANN, Alessandra Elena, 4070 Basel (CH); VOLKOVA-VOLKMAR, Ekaterina, 4070 Basel (CH); CHENG, Wei-Yi, Little Falls, NJ 07424 (US)
(74) Representative: Boult Wade Tennant LLP
(86) International application number: PCT/US2020/051028
(87) International publication number: WO 2021/055443

(56) References cited:
- US-A1- 2014 378 508
- US-A1- 2016 319 355
- US-A1- 2018 206 775
- US-A1- 2019 110 754
- US-A1- 2019 200 915
- US-B1- 8 845 557
- SHARMA VINOD ET AL: "SPARK: Personalized Parkinson Disease Interventions through Synergy between a Smartphone and a Smartwatch", 22 June 2014, SAT 2015 18TH INTERNATIONAL CONFERENCE, AUSTIN, TX, USA, SEPTEMBER 24-27, 2015; [LECTURE NOTES IN COMPUTER SCIENCE; LECT.NOTES COMPUTER], SPRINGER, BERLIN, HEIDELBERG, PAGE(S) 103 - 114, ISBN: 978-3-540-74549-5, XP047341717
- STAMATE C ET AL: "Deep learning Parkinson's from smartphone data", 2017 IEEE INTERNATIONAL CONFERENCE ON PERVASIVE COMPUTING AND COMMUNICATIONS (PERCOM), IEEE, 13 March 2017 (2017-03-13), pages 31 - 40, XP033091667, DOI: 10.1109/PERCOM.2017.7917848
- TSIOURIS KOSTAS M ET AL: "PD_Manager: an mHealth platform for Parkinson's disease patient management", HEALTHCARE TECHNOLOGY LETTERS, vol. 4, no. 3, 23 May 2017 (2017-05-23), pages 102 - 108, XP006076123, DOI: 10.1049/HTL.2017.0007
- ALVARADO-BOLANOS ALONSO ET AL: "Convergent validation of EQ-5D-5L in patients with Parkinson's disease", JOURNAL OF NEUROLOGICAL SCIENCES, ELSEVIER SCIENTIFIC PUBLISHING CO, AMSTERDAM, NL, vol. 358, no. 1, 7 August 2015 (2015-08-07), pages 53 - 57, XP029321606, ISSN: 0022-510X, DOI: 10.1016/J.JNS.2015.08.010
- MARTINEZ-MARTIN P ET AL: "Relationship between the MDS-UPDRS domains and the health-related quality of life of Parkinson's disease patients", EUROPEAN JOURNAL OF NEUROLOGY, RAPID SCIENCE PUBLISHERS, GB, vol. 21, no. 3, 21 January 2014 (2014-01-21), pages 519 - 524, XP072030507, ISSN: 1351-5101, DOI: 10.1111/ENE.12349
- GARCIA-GORDILLO MIGUEL ANGEL ET AL: "Validation and comparison of 15-D and EQ-5D-5L instruments in a Spanish Parkinson's disease population sample", QUALITY OF LIFE RESEARCH, SPRINGER INTERNATIONAL PUBLISHING, CHAM, vol. 23, no. 4, 22 November 2013 (2013-11-22), pages 1315 - 1326, XP035358605, ISSN: 0962-9343, [retrieved on 20131122], DOI: 10.1007/S11136-013-0569-4

## Description

### CROSS REFERENCE TO RELATED APPLICATION

This application claims priority to United States Provisional Application No. 62/901,461 filed September 17, 2019.

### FIELD

Aspects described herein generally relate to digital health tools for medical diagnostics and analytics for use with patients diagnosed with a movement disorder, e.g., Parkinson's disease. Further aspects described herein analyze the association between passive sensor data and health status measurements. Other aspects described herein analyze the association between passive and/or active sensor data and quality of life measurements. Still other aspects described herein analyze the association between passive sensor data and/or active sensor data and disease severity ratings for patients with Parkinson's disease. Additional aspects described herein analyze the reliability and validity of (passively and/or actively) monitored daily motor behavior, e.g., using sensor data from wearable devices and/or smartphones.

### BACKGROUND

Parkinson's disease occurs when brain cells that make dopamine, a chemical that coordinates movement, cease functioning or die. Because Parkinson's disease may cause tremors, slowness, stiffness, and walking and balance problems, it is called a "movement disorder." The disease may include additional side effects such as constipation, depression, memory problems, and other non-movement related symptoms. Unfortunately, Parkinson's disease is progressive with symptoms that slowly worsen over time. Parkinson's disease affects nearly one million people in the United States and more than six million people worldwide.

Presently, there is no definitive test for Parkinson's disease. Typically neurologists, physicians trained in nervous system conditions, will diagnose Parkinson's disease based upon a patient's medical history, review of the patient's signs and symptoms, and a neurological and physical examination. Neurologists may conduct other tests such as a specific single-photon emission computerized tomography scan called a dopamine transporter scan to confirm a diagnosis of Parkinson's disease. Other imaging tests, such as MRI, CT, ultrasound of the brain, and PET scans, may also be used to help rule out other disorders. Symptoms and neurologic examination, however, ultimately determine the correct diagnosis.

Since Parkinson's disease is progressive, early diagnosis and early intervention with medication is desired to slow and manage the disease. Experts have developed additional criteria for tracking the progression of Parkinson's disease through several health surveys used to measure a generic health and quality of life of a Parkinson's disease patient.

The severity of motor impairment among individuals with Parkinson's disease (PD) impacts their perceived quality of life (QoL). More accurate ways to diagnose and assess disease progression are needed to assist those with Parkinson's (and/or other motor diseases) control their symptoms and maintain quality of life.
US Patent Publication US 2018/206775 describes a smartphone based platform that can be used to objectively and remotely measure aspects related to PD (e.g., voice, balance, dexterity, gait, and reaction time), activities of daily living, and PD medicine response. The described platform includes a unified PD-specific remote monitoring platform that incorporates both active and passive tests to provide high frequency monitoring of symptoms and activities of daily living related to PD and medicine response.
US Patent Publication 2019/110754 describes a system and method of diagnosing and monitoring neurological disorders in a patient. The system may comprise a plurality of sensors, a collection of trained machine learning based diagnostic and monitoring tools, and an output device. The plurality of sensors may collect data relevant to neurological disorders.
In conference proceedings of "Sat 2015 18th International Conference, Austin, TX, USA, September 24-27, 2015" dated 22 June 2014, page 103, an article by Sharma *et al.* describes personalised Parkinson disease interventions through synergy between a smartphone and a smartwatch.
In conference proceedings of "2017 IEEE International Conference on Pervasive Computing and Communications (PERCOM)" dated 13 March 2017, pages 31-40, an article by Stamate *et al.* describes deep learning in relation to Parkinson's disease from smartphone data.
An article by Tsiouris et al. in "Healthcare Technology Letters" dated 23 May 2017, vol. 4, no. 3, pages 102-108, discusses an mHealth platform for Parkinson's disease patient management.
Further methods for assessing Parkinson patients are known from ALVARADO-BOLANOS ALONSO ET AL: "Convergent validation of EQ-5D-5L in patients with Parkinson's disease", DOI: 10.1016/ J.JNS.2015.08.010, MARTINEZ-MARTIN P ET AL: "Relationship between the MDS-UPDRS domains and the health-related quality of life of Parkinson's disease patients", ISSN: 1351-5101, DOI: 10.1111/ENE.12349, GARCIA-GORDILLO MIGUEL ANGEL ET AL: "Validation and comparison of 15-D and EQ-5D-5L instruments in a Spanish Parkinson's disease population sample", ISSN: 0962-9343, DOI: 10.1007/511136-013-0569-4, and US2019/200915-A1.

### SUMMARY OF THE INVENTION

The invention is defined by the claims. The following presents a simplified summary of various aspects described herein. This summary is not an extensive overview, and is not intended to identify key or critical elements or to delineate the scope of the claims. The following summary merely presents some concepts in a simplified form as an introductory prelude to the more detailed description provided below.

Smartphone-based and smartwatch-based sensors-and the related mobile applications disclosed herein-represent a reliable and valid means to estimate motor impairment in the daily life of a PD patient. The disclosures herein describe systems and methods for assessing motor symptoms in PD patients through passively and/or actively acquired sensor data of motor behaviors and generating motor system assessments. Movement data comprising passive movement data and/or active motor performance data collected from Parkinson's disease patients are associated with EQ-5D-5L, PDQ-39, and/or MDS-UPDRS health status measurement data. Accordingly, the collected passive and/or active motor movement data can be used to more accurately predict PD patient QoL, health status, and disease severity. The generated motor system assessment may be used to provide neurologists and other health professionals with more accurate treatment recommendations and disease management protocols rather than using patient surveys alone.

To overcome limitations in the prior art described above, and to overcome other limitations that will be apparent upon reading and understanding the present specification, aspects described herein are directed to methods for assessing motor symptoms in Parkinson patients may include obtaining, from a sensor, patient movement data. The movement data may include passive movement. In some examples, the method may include determining that the patient movement data is impaired or not impaired, and generating, based on the patient movement data, a motor system assessment. The motor system assessment may be impaired or not impaired. In some examples, the sensor may be within a mobile telephone or a smart watch. In other examples, the patient movement data may include at least one of gesture power, step power, gait span, pathological hand tremor frequency, gesture time, turn speed, and gesture span duration. In still other examples, the motor system assessment is highly correlative to EQ-5D-5L health status measurement data. Gesture power may correlate or be indicative of EQ-5D-5L Mobility data, gesture power, step power, gait span, and pathological hand tremor frequency may be indicative of EQ-5D-5L Self-Care data, gesture power, step power, and gesture time data may correlate to or be indicative of EQ-5D-5L, Usual Activities data, gesture power and/or turn speed may be indicative of EQ-5D-5L Pain/Discomfort data, and gesture time, gesture span duration, and gait span data may be indicative of EQ-5D-5L Anxiety/Depression data. In yet other examples, the method may further include calculating, based on a patient-population movement data in which the movement data comprises passive movement data, a threshold value defining the motor system assessment as impaired or not impaired. In other examples, the method may further include calculating a patient movement score, comparing the patient movement score to the threshold value, and determining if the motor system assessment is impaired or not impaired. In other examples, the method may further include displaying a command, at a server or mobile device, that may be no treatment for a not impaired motor system assessment, or displaying a command that may be to initiate a treatment for an impaired motor system assessment. In still other examples, the method may further include calculating the treatment based on the motor system assessment in which the treatment may be delivering a therapeutically effective amount of a drug to the Parkinson's disease patient.

Systems disclosed herein for assessing motor symptoms in Parkinson patients may include a display, a sensor, a processor, and a memory that may be in communication with the processor storing instructions that, when executed by the processor, may cause the system to obtain, from the sensor, patient movement data comprising passive movement data, determine that the patient movement data may be impaired or not impaired, and generate, based on the patient movement data, a motor system assessment that may be impaired or not impaired. In some examples, the system may include a mobile telephone or a smart watch. According to the invention, the patient passive movement data include gesture power, step power, gait span, pathological hand tremor frequency, gesture time, turn speed, and gesture span duration. In certain examples, the motor system assessment may be highly correlative to EQ-5D-5L health status measurement data, and gesture power may be indicative of EQ-5D-5L Mobility data, gesture power, step power, gait span, and pathological hand tremor frequency may be indicative of EQ-5D-5L Self-Care data, gesture power, step power, and gesture time data may be indicative of EQ-5D-5L Usual Activities data, gesture power and/or turn speed may be indicative of EQ-5D-5L Pain/Discomfort data, gesture time, gesture span duration, and gait span data may indicative of EQ-5D-5L Anxiety/Depression data. In yet other examples, the instructions, when read by the processor, may further cause the system to calculate, based on a patient-population movement data in which the movement data may be passive movement data, a threshold value defining the motor system assessment as impaired or not impaired. In other examples, the system may include calculating a patient movement score, comparing the patient movement score to the threshold value, and determining if the motor system assessment is impaired or not impaired. In still other examples, the instructions, when read by the processor, may cause the system to further display a command, at a server or other mobile device, in which the command may be no treatment for a not impaired motor system assessment, or display a command to initiate a treatment for an impaired motor system assessment, and may calculate the treatment based on the motor system assessment in which the treatment may be delivering a therapeutically effective amount of a drug to the Parkinson's disease patient.

In another embodiment disclosed herein, a non-transitory machine-readable medium storing instructions may, when executed by one or more processors, cause the one or more processors to perform steps to include obtain, from a sensor, patient movement data including passive movement data, determine that the patient movement data is impaired or not impaired, and generate, based on the patient movement data, a motor system assessment that is impaired or not impaired. According to the invention, the patient movement data include gesture power, step power, gait span, pathological hand tremor frequency, gesture time, turn speed, and gesture span duration. In yet other examples, the instructions may further cause the one or more processors to perform steps to include calculate, based on a patient-population movement data, a threshold value defining the motor system assessment as impaired or not impaired, calculate a patient movement score, compare the patient movement score to the threshold value, and determine if the motor system assessment is impaired or not impaired. In yet other examples, the instructions may further cause the one or more processors to perform steps to include display a command that is no treatment for a not impaired motor system assessment, or display a command that is to initiate a treatment for an impaired motor system assessment. In still another example, the instructions may further cause the one or more processors to perform steps to include calculate the treatment based on the motor system assessment in which the treatment is delivering a therapeutically effective amount of a drug to the Parkinson's disease patient.

Additional methods disclosed herein for assessing motor symptoms in Parkinson patients may include obtaining, from a sensor, such as a mobile telephone or a smart watch, patient movement data that may include active performance data, determining that the patient movement data is impaired or not impaired, and generating, based on the patient movement data, a motor system assessment, that is impaired or not impaired. In some examples, the patient active movement data may include at least one of drawing a shape, dexterity, hand turning, speech, phonation, postural tremor, rest tremor, balance, U-turn, and cognitive test (Symbol Digit Modalities Test). In other examples, the motor system assessment may be highly correlative to quality of life (PDQ-39) measurements, and drawing a shape, dexterity, or hand turning may be indicative of PDQ-39 Activities of Daily Living, Communication, or Mobility data, speech may be indicative of PDQ-39 Communication data, and U-turn may be indicative of PDQ-39 Mobility data. In still other examples, the method may also include calculating, based on a patient-population movement data including active performance data, a threshold value defining the motor system assessment as impaired or not impaired. In some examples, the methods may also include calculating a patient performance score, comparing the patient performance score to the threshold value, and determining if the motor system assessment may be impaired or not impaired. In yet other examples, the method may include displaying a command that may be no treatment for a not impaired motor system assessment, or displaying a command that may be to initiate a treatment for an impaired motor system assessment. In yet another example, the method may also include calculating the treatment based on the motor system assessment in which the treatment may be delivering a therapeutically effective amount of a drug to the patient.

Additional systems disclosed herein for assessing motor symptoms in Parkinson patients may include a display, a sensor, a processor, and a memory that may be in communication with the processor storing instructions that, when executed by the processor, cause the system to obtain, from the sensor, patient movement data including active performance data. In some examples, the system may then determine that the patient movement data may be impaired or not impaired, and generate, based on the patient movement data, a motor system assessment that may be impaired or not impaired. In certain examples, the system may include a mobile telephone or a smart watch. In other examples, the patient active movement data may include at least one of drawing a shape, dexterity, hand turning, speech, phonation, postural tremor, rest tremor, balance, U-turn, and cognitive test (Symbol Digit Modalities Test). In still other examples, the motor system assessment may be highly correlative to quality of life (PDQ-39) measurements, and drawing a shape, dexterity, or hand turning may be indicative of PDQ-39 Activities of Daily Living, Communication, or Mobility data, speech may be indicative of PDQ-39 Communication data, and U-turn may be indicative of PDQ-39 Mobility data. In yet other examples, the instructions, when read by the processor, may further cause the system to calculate, based on a patient-population movement data in which the movement data may include active performance data, a threshold value defining the motor system assessment as impaired or not impaired. In other examples, the system may include calculating a patient performance score, comparing the patient performance score to the threshold value, and determining if the motor system assessment is impaired or not impaired. In still other examples, the instructions, when read by the processor, may cause the system to further display a command, at a server or other mobile device, in which the command may be no treatment for a not impaired motor system assessment, or display a command to initiate a treatment for an impaired motor system assessment, and may calculate the treatment based on the motor system assessment in which the treatment may be delivering a therapeutically effective amount of a drug to the Parkinson's disease patient.

In another embodiment disclosed herein, a non-transitory machine-readable medium storing instructions may, when executed by one or more processors, cause the one or more processors to perform steps to include obtain, from a sensor, patient movement data that may include active performance data, determine that the patient movement data is impaired or not impaired, and generate, based on the patient movement data, a motor system assessment that is impaired or not impaired. In other examples, the patient movement data may include at least one of drawing a shape, dexterity, hand turning, speech, phonation, postural tremor, rest tremor, balance, U-turn, and cognitive test (Symbol Digit Modalities Test). In yet other examples, the instructions may further cause the one or more processors to perform steps to include calculate, based on a patient-population movement data that includes active performance data, a threshold value defining the motor system assessment as impaired or not impaired, calculate a patient active performance score, compare the patient active performance score to the threshold value, and determine if the motor system assessment is impaired or not impaired. In yet other examples, the instructions may further cause the one or more processors to perform steps to include display a command that is no treatment for a not impaired motor system assessment, or display a command that is to initiate a treatment for an impaired motor system assessment. In still another example, the instructions may further cause the one or more processors to perform steps to include calculate the treatment based on the motor system assessment in which the treatment is delivering a therapeutically effective amount of a drug to the Parkinson's disease patient.

Other methods disclosed herein for assessing motor symptoms in Parkinson patients may include obtaining, from a sensor, such as a mobile telephone or a smart watch, patient movement data that may include passive movement data and/or active performance data, determining that the patient movement data is impaired or not impaired, and generating, based on the patient movement data, a motor system assessment, that is impaired or not impaired. In some examples, the patient active performance data may include at least one of drawing a shape, dexterity, hand turning, speech, phonation, postural tremor, rest tremor, balance, U-turn, and cognitive test (Symbol Digit Modalities Test), and the patient passive movement data may include at least one of gait, arm swing/tremor, and mobility/sociability. In other examples, the motor system assessment may be highly correlative to disease severity ratings (MDS-UPDRS), and rest tremor may be indicative of MDS-UPDRS Rest Tremor Amplitude data, postural tremor may be indicative of MDS-UPDRS Rest Tremor Amplitude data, balance may be indicative of MDS-UPDRS Consistency of Rest Tremor data, hand turning may be indicative of MDS-UPDRS Hand Movements data, dexterity may be indicative of MDS-UPDRS Finger Tapping data, drawing a shape may be indicative of MDS-UPDRS Handwriting data, U-turn may be indicative of MDS-UPDRS Body Bradykinesia data, cognitive test (Symbol Digit Modalities Test) may be indicative of MDS-UPDRS Cognitive Impairment data, phonation may be indicative of MDS-UPDRS Speech Problems data, speech may be indicative of MDS-UPDRS Saliva and Drooling data, and gait may be indicative of MDS-UPDRS Body Bradykinesia data. In still other examples, the method may also include calculating, based on a patient-population movement data including passive movement data and/or active performance data, a threshold value defining the motor system assessment as impaired or not impaired. In some examples, the methods may also include calculating a patient movement/performance score, comparing the patient movement/performance score to the threshold value, and determining if the motor system assessment may be impaired or not impaired. In yet other examples, the method may include displaying a command that may be no treatment for a not impaired motor system assessment, or displaying a command that may be to initiate a treatment for an impaired motor system assessment. In yet another example, the method may also include calculating the treatment based on the motor system assessment in which the treatment may be delivering a therapeutically effective amount of a drug to the patient.

Other systems disclosed herein for assessing motor symptoms in Parkinson patients may include a display, a sensor, a processor, and a memory that may be in communication with the processor storing instructions that, when executed by the processor, cause the system to obtain, from the sensor, patient movement data including passive movement data and/or active performance data. In some examples, the system may then determine that the patient movement data may be impaired or not impaired, and generate, based on the patient movement data, a motor system assessment that may be impaired or not impaired. In certain examples, the system may include a mobile telephone or a smart watch. In other examples, the patient active performance data may include at least one of drawing a shape, dexterity, hand turning, speech, phonation, postural tremor, rest tremor, balance, U-turn, and cognitive test (Symbol Digit Modalities Test), and the patient passive movement data may include at least one of gait, arm swing/tremor, and mobility/sociability. In other examples, the motor system assessment may be highly correlative to disease severity ratings (MDS-UPDRS), and rest tremor may be indicative of MDS-UPDRS Rest Tremor Amplitude data, postural tremor may be indicative of MDS-UPDRS Rest Tremor Amplitude data, balance may be indicative of MDS-UPDRS Consistency of Rest Tremor data, hand turning may be indicative of MDS-UPDRS Hand Movements data, dexterity may be indicative of MDS-UPDRS Finger Tapping data, drawing a shape may be indicative of MDS-UPDRS Handwriting data, U-turn may be indicative of MDS-UPDRS Body Bradykinesia data, cognitive test (Symbol Digit Modalities Test) may be indicative of MDS-UPDRS Cognitive Impairment data, phonation may be indicative of MDS-UPDRS Speech Problems data, speech may be indicative of MDS-UPDRS Saliva and Drooling data, and gait may be indicative of MDS-UPDRS Body Bradykinesia data. In yet other examples, the instructions, when read by the processor, may further cause the system to calculate, based on a patient-population movement data in which the movement data may include passive movement data and/or active performance data, a threshold value defining the motor system assessment as impaired or not impaired. In other examples, the system may include calculating a patient movement/performance score, comparing the patient movement/performance score to the threshold value, and determining if the motor system assessment is impaired or not impaired. In still other examples, the instructions, when read by the processor, may cause the system to further display a command, at a server or other mobile device, in which the command may be no treatment for a not impaired motor system assessment, or display a command to initiate a treatment for an impaired motor system assessment, and may calculate the treatment based on the motor system assessment in which the treatment may be delivering a therapeutically effective amount of a drug to the Parkinson's disease patient.

In another embodiment disclosed herein, a non-transitory machine-readable medium storing instructions may, when executed by one or more processors, cause the one or more processors to perform steps to include obtain, from a sensor, patient movement data that may include passive movement data and/or active performance data, determine that the patient movement data is impaired or not impaired, and generate, based on the patient movement data, a motor system assessment that is impaired or not impaired. In other examples, the patient active performance data may include at least one of drawing a shape, dexterity, hand turning, speech, phonation, postural tremor, rest tremor, balance, U-turn, and cognitive test (Symbol Digit Modalities Test), and the patient passive movement data may include at least one of gait, arm swing/tremor, and mobility/sociability. In other examples, the motor system assessment may be highly correlative to disease severity ratings (MDS-UPDRS), and rest tremor may be indicative of MDS-UPDRS Rest Tremor Amplitude data, postural tremor may be indicative of MDS-UPDRS Rest Tremor Amplitude data, balance may be indicative of MDS-UPDRS Consistency of Rest Tremor data, hand turning may be indicative of MDS-UPDRS Hand Movements data, dexterity may be indicative of MDS-UPDRS Finger Tapping data, drawing a shape may be indicative of MDS-UPDRS Handwriting data, U-turn may be indicative of MDS-UPDRS Body Bradykinesia data, cognitive test (Symbol Digit Modalities Test) may be indicative of MDS-UPDRS Cognitive Impairment data, phonation may be indicative of MDS-UPDRS Speech Problems data, speech may be indicative of MDS-UPDRS Saliva and Drooling data, and gait may be indicative of MDS-UPDRS Body Bradykinesia data. In yet other examples, the instructions may further cause the one or more processors to perform steps to include calculate, based on a patient-population movement data that includes passive movement data and/or active performance data, a threshold value defining the motor system assessment as impaired or not impaired, calculate a patient movement/performance score, compare the patient movement/performance score to the threshold value, and determine if the motor system assessment is impaired or not impaired. In yet other examples, the instructions may further cause the one or more processors to perform steps to include display a command that is no treatment for a not impaired motor system assessment, or display a command that is to initiate a treatment for an impaired motor system assessment. In still another example, the instructions may further cause the one or more processors to perform steps to include calculate the treatment based on the motor system assessment in which the treatment is delivering a therapeutically effective amount of a drug to the Parkinson's disease patient.

These features, along with many others, are discussed in greater detail below.

### BRIEF DESCRIPTION OF THE DRAWINGS

A more complete understanding of aspects described herein and the advantages thereof may be acquired by referring to the following description in consideration of the accompanying drawings, in which like reference numbers indicate like features, and wherein:
FIG. 1 illustrates a custom computing system and architecture which may be used to implement one or more illustrative aspects described herein.
FIG. 2A is a flowchart conceptually illustrating a process for analyzing active and passive movement digital biomarker data and determining motor system assessment according to one or more illustrative aspects described herein.
FIG. 2B is a flowchart conceptually illustrating a process for active and passive testing of digital biomarkers according to the systems and methods disclosed herein.
FIG. 3A graphically depicts a sample study distribution of EQ-5D-5L quality of life domain scores across the five categories of Anxiety/Depression, Mobility, Pain/Discomfort, Self-Care, and Usual Activities.
FIG. 3B displays the EQ-5D-5L domain scores graphically depicted in FIG. 3A.
FIG. 4A depicts data distribution collected by a digital biomarker sensor application and related movements correlated to the EQ-5D-5L category of Self-Care graphically displayed in FIG. 3A, according to the systems and methods disclosed herein.
FIG. 4B depicts data distribution collected by a digital biomarker sensor application and related movements correlated to the EQ-5D-5L category of Mobility graphically displayed in FIG. 3A, according to the systems and methods disclosed herein.
FIG. 4C depicts data distribution collected by a digital biomarker sensor application and related movements correlated to the EQ-5D-5L, category of Usual Activities graphically displayed in FIG. 3A, according to the systems and methods disclosed herein.
FIG. 4D depicts data distribution collected by a digital biomarker sensor application and related movements correlated to the EQ-5D-5L category of Pain/Discomfort graphically displayed in FIG. 3A, according to the systems and methods disclosed herein.
FIG. 4E depicts data distribution collected by a digital biomarker sensor application and related movements correlated to the EQ-5D-5L category of Anxiety/Depression graphically displayed in FIG. 3A, according to the systems and methods disclosed herein.
FIG. 5A depicts correlations between PDQ-39 quality of life dimension scores and selected passive movements obtained by a digital biomarker sensor application, according to the systems and methods disclosed herein.
FIG. 5B graphically depicts the distributions of PDQ-39 dimension scores at screening visit (boxplots).
FIG. 5C illustrates the active testing suite of the updated version of the digital biomarker sensor application, according to the systems and methods disclosed herein.
FIG. 5D depicts correlations between PDQ-39 quality of life dimension scores and selected active sensor features obtained by an updated version of the digital biomarker sensor application, according to the systems and methods disclosed herein.
FIG. 5E depicts data distribution of test patient's and related demographics who utilized the active testing suite of the updated version of the digital biomarker sensor application of FIG. 5C, according to the systems and methods disclosed herein. Distributions are displayed as mean ± SD.
FIGS. 5F-5I graphically depict the active sensor suite differences between impaired and non-impaired individuals on PDQ-39 items, according to the systems and methods disclosed herein.
FIG. 6A depicts correlations between MDS-UPDRS composite scores and selected passive movements obtained by a digital biomarker sensor application, according to the systems and methods disclosed herein.
FIG. 6B depicts Spearman correlations and Mann-Whitney tests for impairment correlations for MDS-UPDRS composite scores and selected passive movements obtained by a digital biomarker sensor application, according to the systems and methods disclosed herein.
FIG. 6C graphically depicts bradykinesia total distribution for test subject correlation to MDS-UPDRS composite scores and selected passive movements obtained by a digital biomarker sensor application, according to the systems and methods disclosed herein.
FIGS. 6D-6E graphically depict test subject sedentary upper limb movements correlated to MDS-UPDRS Apathy and Pron/Sup Movement of Left Hand scores obtained by a digital biomarker sensor application, according to the systems and methods disclosed herein.
FIGS. 6F-6H graphically depict test subject lower limb movements correlated to MDS-UPDRS Gait, Freezing, and Get Out of Bed/Car/Deep Chair scores obtained by a digital biomarker sensor application, according to the systems and methods disclosed herein.
FIG. 7A illustrates the combined active and passive testing suite of an updated version of the digital biomarker sensor application, according to the systems and methods disclosed herein.
FIG. 7B depicts data distribution of test patient's and related demographics who utilized the combined active and passive testing suite of the updated version of the digital biomarker sensor application of FIG. 7A, according to the systems and methods disclosed herein.
FIG. 7C graphically depicts adherence results of collected active and passive data obtained by the updated digital biomarker sensor application of FIG. 7A, according to the systems and methods disclosed herein.
FIG. 7D graphically depicts test-retest reliability of active data obtained by the updated digital biomarker sensor application of FIG. 7A, according to the systems and methods disclosed herein.
FIG. 7E depicts the predicted sensor features that significantly correlated with corresponding MDS-UPDRS items at baseline.
FIGS. 7F-7H graphically depict example relationships between sensor features and corresponding MDS-UPDRS item scores, according to the systems and methods disclosed herein.

### DETAILED DESCRIPTION

In the following description of the various embodiments, reference is made to the accompanying drawings, which form a part hereof, and in which is shown by way of illustration various embodiments in which aspects described herein may be practiced. It is to be understood that other embodiments may be utilized and structural and functional modifications may be made without departing from the scope of the described aspects and embodiments. Aspects described herein are capable of other embodiments and of being practiced or being carried out in various ways. Also, it is to be understood that the phraseology and terminology used herein are for the purpose of description and should not be regarded as limiting. Rather, the phrases and terms used herein are to be given their broadest interpretation and meaning. The use of "including" and "comprising" and variations thereof is meant to encompass the items listed thereafter and equivalents thereof as well as additional items and equivalents thereof. The use of the terms "mounted," "connected," "coupled," "positioned," "engaged" and similar terms, is meant to include both direct and indirect mounting, connecting, coupling, positioning and engaging.

As a general introduction to the subject matter described herein, gesture recognition using a smartphone, smartwatch, wearable, or similar device may be used to assess motor signs and symptom severity in individuals diagnosed with Parkinson's disease, and subsequently correlated to quality of life measure (PDQ-39), and/or health status (EQ-5D-5L), and/or disease severity ratings (MDS-UPDRS) for patients with Parkinson's Disease.

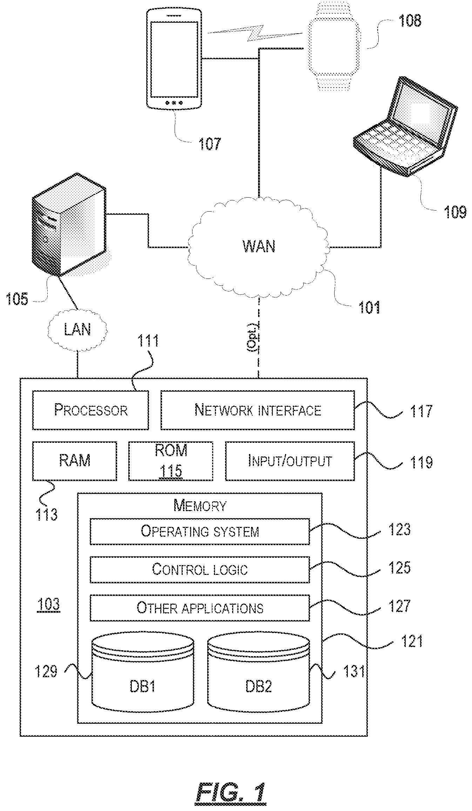

FIG. 1 illustrates one example of a custom network architecture and data processing devices that may be used to implement one or more illustrative aspects described herein. Various network nodes 103, 105, 107, 108 and 109 may be interconnected via a wide area network (WAN) 101, such as the Internet. Other networks may also or alternatively be used, including private intranets, corporate networks, LANs, wireless networks, personal networks (PAN), and the like. Network 101 is for illustration purposes and may be replaced with fewer or additional computer networks. A local area network (LAN) may have one or more of any known LAN topology and may use one or more of a variety of different protocols, such as Ethernet. Devices 103, 105, 107, 108, 109 and other devices (not shown) may be connected to one or more of the networks via twisted pair wires, coaxial cable, fiber optics, radio waves or other communication media.

The term "network" as used herein and depicted in the drawings refers not only to systems in which remote storage devices are coupled together via one or more communication paths, but also to stand-alone devices that may be coupled, from time to time, to such systems that have storage capability. Consequently, the term "network" includes not only a "physical network" but also a "content network," which is comprised of the data-attributable to a single entity-which resides across all physical networks.

The components may include data server 103, web server 105, and client devices 107-109. Data server 103 provides overall access, control and administration of databases and control software for performing one or more illustrative aspects described herein. Data server 103 may be connected to web server 105 through which users interact with and obtain data as requested. Alternatively, data server 103 may act as a web server itself and be directly connected to the Internet (in which case device 105 is not needed). Data server 103 may be connected to web server 105 through the network 101 (e.g., the Internet), via direct or indirect connection, or via some other network. Users may interact with the data server 103 using remote computers 107-109, e.g., using an application, mobile app, wearable app, or web browser to connect to the data server 103 via one or more externally exposed web sites and/or web services hosted by web server 105. Client computers 107-109 may be used in concert with data server 103 to access data stored therein, or may be used for other purposes. For example, from client device 107, 108 a user may access web server 105 using an Internet browser, as is known in the art, or by executing a software application that communicates with web server 105 and/or data server 103 over a computer network (such as the Internet).

Servers and applications may be combined on the same physical machines, and retain separate virtual or logical addresses, or may reside on separate physical machines. FIG. 1 illustrates just one example of a network architecture that may be used, and those of skill in the art will appreciate that the specific network architecture and data processing devices used may vary, and are secondary to the functionality that they provide, as further described herein. For example, services provided by web server 105 and data server 103 may be combined on a single server.

Each component 103, 105, 107, 108, 109 may be any type of known computer, server, or data processing device accessing a web-based implementation, or a custom device integrated with special purpose software, as further described herein, e.g., mobile or smartphone, smartwatch, or other wearable device. Data server 103, e.g., may include a processor 111 controlling overall operation of the data server 103. Data server 103 may further include RAM 113, ROM 115, network interface 117, input/output interfaces 119 (e.g., keyboard, mouse, display, printer, etc.), and memory 121. I/O 119 may include a variety of interface units and drives for reading, writing, displaying, and/or printing data or files. Memory 121 may further store operating system software 123 for controlling overall operation of the data processing device 103, control logic 125 for instructing data server 103 to perform aspects described herein, and other application software 127 providing secondary, support, and/or other functionality which may or may not be used in conjunction with other aspects described herein. The control logic may also be referred to herein as the data server software 125. Functionality of the data server software may refer to operations or decisions made automatically based on rules coded into the control logic, made manually by a user providing input into the system, and/or a combination of automatic processing based on user input (e.g., queries, data updates, etc.).

Memory 121 may also store data used in performance of one or more aspects described herein, including a first database 129 and a second database 131. In some embodiments, the first database may include the second database (e.g., as a separate table, report, etc.). That is, the information can be stored in a single database, or separated into different logical, virtual, or physical databases, depending on system design. Devices 105, 107, 109 may have similar or different architecture as described with respect to device 103. Those of skill in the art will appreciate that the functionality of data processing device 103 (or device 105, 107, 109) as described herein may be spread across multiple data processing devices, for example, to distribute processing load across multiple computers, to segregate transactions based on geographic location, user access level, quality of service (QoS), etc.

One or more aspects described herein may be embodied in computer-usable or readable data and/or computer-executable instructions, such as in one or more program modules, executed by one or more computers or other devices as described herein. Generally, program modules include routines, programs, objects, components, data structures, etc. that perform particular tasks or implement particular abstract data types when executed by a processor in a computer or other device. The modules may be written in a source code programming language that is subsequently compiled for execution, or may be written in a scripting language such as (but not limited to) HTML or XML. The computer executable instructions may be stored on a computer readable medium such as a hard disk, optical disk, removable storage media, solid state memory, RAM, etc. As will be appreciated by one of skill in the art, the functionality of the program modules may be combined or distributed as desired in various embodiments. In addition, the functionality may be embodied in whole or in part in firmware or hardware equivalents such as integrated circuits, field programmable gate arrays (FPGA), and the like. Particular data structures may be used to more effectively implement one or more aspects, and such data structures are contemplated within the scope of computer executable instructions and computer-usable data described herein.

Aspects described herein generally relate to digital health tools for medical diagnostics and analytics for use with patients diagnosed with a movement disorder, e.g., Parkinson's disease. Further aspects described herein may be used to assess motor signs and symptom severity in individuals with Parkinson's disease, and to analyze the association between passive sensor data and health status (EQ-5D-5L) measurements. Other aspects described herein may be used to assess motor signs and symptom severity in individuals with Parkinson's disease, and to analyze the association between passive/active sensor data quality of life (QoL) (PDQ-39) measurements. Additional aspects described herein may be used to assess motor signs and symptom severity in individuals with Parkinson's disease, and to analyze the association between active/passive sensor data and disease severity ratings (MDS-UPDRS) for patients with Parkinson's disease. Additional aspects described herein analyze the reliability and validity of (passively and/or actively) monitored daily motor behavior, e.g., using sensor data from wearable devices and/or smartphones to assess motor signs and symptom severity in individuals with Parkinson's disease. Motor functioning in daily life is one of the most ecologically valid reflections of motor disease severity, but is difficult to quantify objectively.

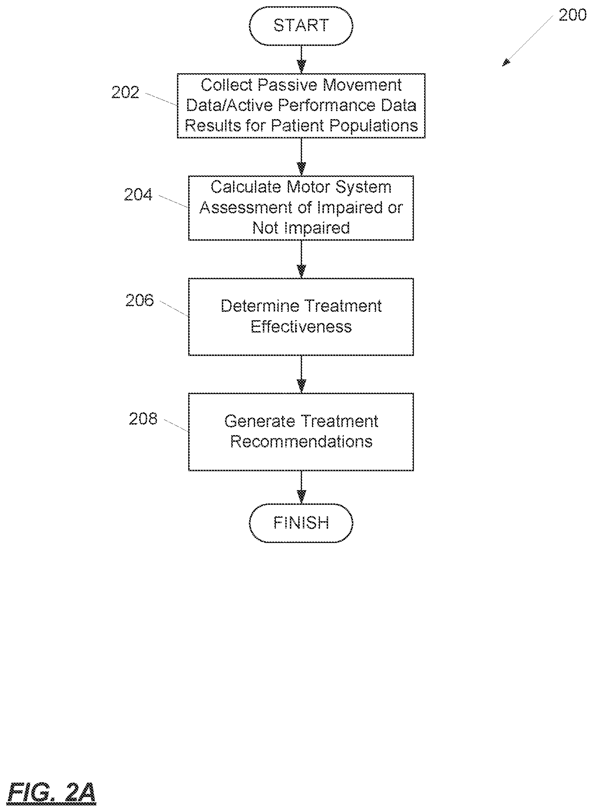

FIG. 2A is a flowchart conceptually illustrating a process for analyzing active and passive movement digital biomarker data and determining a motor system assessment according to one or more illustrative aspects described herein. Some or all of the steps of process 200 may be performed using one or more computing devices as described herein. In a variety of embodiments, some or all of the steps described below may be combined and/or divided into sub-steps as appropriate.

At step 202, passive movement data and/or active performance data for one or more patient populations may be collected. Generally, "movement data" may include passive movement data and/or active performance data. The data may be obtained from a variety of sources, such as wearable devices and/or smartphones, or other mobile devices used by the patients as described herein. The movement data may also include a variety of characteristics for each patient, such as the user's age, gender, etc. The patients of a particular patient population may have one or more characteristics in common. These characteristics may include, but are not limited to, age, current conditions, diseases, mental capacity, cognitive ability, prior QoL scores, administered treatments, and the like. For example, members of a first patient population may have PD and be under the age of 50, while patients in a second patient population also have PD, but are over the age of 50. In another example, patients in a first patient population may be prescribed a first treatment, while patients in a second patient population may be prescribed a second treatment. However, it should be noted that any number of patient populations may be used and/or any particular combination of characteristics may be used to determine the patient populations. In this way, patient populations may include one or more patients having common characteristics such that patients in different populations may be analyzed and/or compared as appropriate.

At step 204, a motor system assessment may be calculated. An impaired or not impaired motor system assessment may be calculated for each patient in the patient populations using any of a variety of the movement data collection techniques as described herein. In a number of embodiments, the motor system assessment for a particular patient includes collection of movement data over a time series for the patient as described herein. The motor system assessment for a patient may include a prior motor system assessment based on previously collected movement data and/or new movement data collected over time.

At step 206, treatment effectiveness may be determined. The treatment effectiveness may be determined on a per-patient basis and/or on a per-patient population basis as appropriate. The treatment effectiveness for a particular patient may be determined for a patient based on the spatial working memory scores for the patient. The treatment effectiveness for a patient population may be determined based on the motor system assessment for each patient in the patient population. Any of a variety of statistical techniques may be used to determine treatment effectiveness as appropriate. For example, the current motor system assessment for the patient population may be compared to one or more historical motor system assessments for the patient population. The delta between the current motor system assessment and one or more of the historical motor system assessments, a rate of change over time, a moving average of the motor system assessments, and/or any other statistical measure may be used to evaluate the treatment effectiveness. The treatment effectiveness may also be compared across patient populations. For example, a drug may be administered to patient populations divided by age to determine the effectiveness of the drug on the patient's motor system assessment based on the age of the patient. In a second example, a first patient population may be administered a first drug and a second patient population may be administered a placebo. The difference change in motor system assessment between the first patient population and the second patient population may be used to determine the effectiveness of treatments including the first drug. In a third example, a first patient population may be administered a first drug and a second patient population may be administered a second drug. The difference change in motor system assessment between the first patient population and the second patient population may be used to determine which of the first drug and the second drug are more effective at improving PD symptoms. In a fourth example, a first patient population may be administered a drug at a first dosage and a second patient population may be administered the same drug at a second dosage. The difference in improvement (or lack thereof) in motor system assessment for the patient populations may be used to determine the effectiveness of particular dosages of the drug. However, it should be noted that any comparison, such as between patient populations having different conditions affecting motor system assessment, may be used to determine treatment effectiveness as appropriate.

At step 208, treatment recommendations may be generated. The treatment recommendations may be generated based on the effectiveness of the treatments and/or the characteristics of particular patient populations. The treatment recommendations may be generalized and/or targeted towards patients having particular characteristics in common with particular patient populations. For example, if a particular drug is effective in improving motor system assessment for PD patients under the age of 50, the generated treatment recommendation may include a particular dosage for the drug for PD patients under the age of 50. Similarly, if the particular drug is not effective in improving the motor system assessment for PD patients over the age of 50, the treatment recommendation may not include the drug for patients having these characteristics. The treatment recommendations generated for patient populations may be used to recommend and/or administer treatments to particular patients having characteristics in common with the patient populations as described herein.

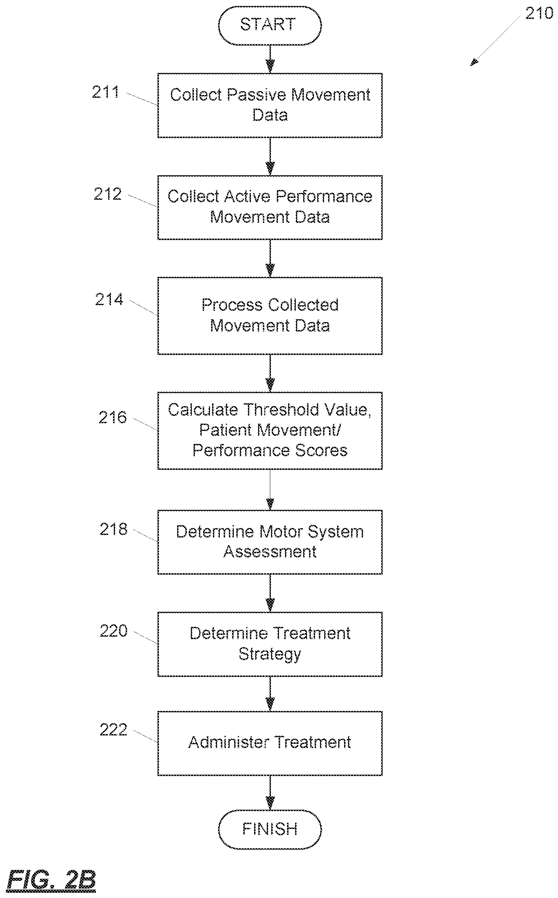

Turning now to FIG. 2B, a flowchart conceptually illustrating a process for active performance and passive testing of digital biomarkers according to the systems and methods disclosed herein is shown. Some or all of the steps of process 210 may be performed using one or more computing devices as described herein. In a variety of embodiments, some or all of the steps described below may be combined and/or divided into sub-steps as appropriate. As previously noted, "movement data" may generally include passive movement data and/or active performance data.

At step 211, passive data may be collected. Passive data may be collected using one or more sensors, such as accelerometers, gyroscopes, magnetometers, and the like, that are located within a mobile device work by a patient. The mobile device can include any computing device typically carried on the patient, such as a mobile phone, smart watch, activity monitor, and the like. The collected passive data can include a variety of movements made by the patient and metrics regarding the movements. These movements and metrics include, but are not limited to, gesture time, gesture power, turns per walking, frequency asymmetry, step power, step frequency variance, gait ratio, turn angle, turn speed, pathological hand tremor (pht) frequency, step frequency, power variance, and any other passive movement described herein. The passive data may be captured during the patient's normal activities.

At step 212, active performance data may be collected. The active performance data may be collected using one or more sensors, such as accelerometers, gyroscopes, magnetometers, and the like, that are located within a mobile device work by a patient. The active performance data may be collected while the patient is engaged in one or more medical tests and/or examinations as described herein. The collected active performance data can include a variety of tasks such as, but not limited to, drawing a shape, dexterity tests, hand tuᵣning, speech tests, phonation tests, postural tremor, rest tremor, balance, U-turn, cognitive Symbol Digit Modalities Tests (SDMT), and any other active performance movement described herein. The active performance data may be collected during specific times that the patient is engaged in the tests and/or examinations.

At step 214, the collected movement data may be processed. The collected movement data may be processed via the mobile device. In other examples, the collected movement data may be uploaded to a server (e.g., cloud, backend server; etc.) and processed by the server. The collected sensor data may be corrected for age and gender. Test-retest reliability may be assessed using intra-class correlation coefficients. In a variety of embodiments, the collected data is aggregated over a particular period. For example, passive movement data can be averaged over a moving seven-day period. Aggregating the collected data may increase the signal-to-noise ratio of the measurements of motor progression for evaluating the patient's health. In a variety of embodiments, the collected data may be in a time domain where a piece of data is coupled with a time that the data was collected. The collected data may be transformed into a frequency domain using any of a variety of techniques, such as a Fourier transform. A variety of noise signatures can be identified and/or subtracted from the frequency domain representation of the collected data. For example, the patient may be a 68 year-old man with a particular hand size and the collected data can be indicative of hand gestures. A noise signal indicative of a hand tremor in a person having a hand similar in size to the patient can be identified. This noise signal can be subtracted from the frequency domain representation in order to remove noise in the captured data caused by the shaking of the patient's hand during the hand gesture. In this way, the frequency domain representation can be de-noised to improve the quality of the measured gesture data in the frequency domain representation of the captured data. The de-noised frequency domain representation of the captured data can then be transformed back into the time domain using any of a variety of techniques, such as an inverse Fourier transform. In this way, the collected data can be processed for analysis in both the frequency domain and/or time domain as appropriate. Further, subtracting noise from the collected data may improve the signal-to-noise ration of the collected data, thereby allowing for improved processing. This may be particularly valuable in determining certain actions, such as gesture power, where unintended motion in the patient's limbs (e.g., tremors) can cause sensors in a mobile device to read a large change in acceleration that are not related to the gesture being performed.

At step 216, a patient-population threshold value, and patient passive movement/active performance scores may be calculated. Again, "movement data" may include passive movement data and/or active performance data. The patient-population threshold value, as determined by a particular patient-population may be used to establish a particular threshold that defines what particular movement or performance scores are defined as impaired or not impaired. The movement and/or performance scores may indicate a baseline quality of life for the patient during their day-to-day activities. In several embodiments, the movement score is calculated based on the collected passive data (i.e., daily activities). At step 216, performance scores may also be calculated based upon the active performance movement data. The active performance score may indicate a baseline level of skill at performing particular tasks. In many embodiments, the active performance scores are calculated based on the active performance movement data. In a number of embodiments, the active performance scores can be calculated based on the movement scores for passive activities that are related to the active performance task. For example, if the active performance task is drawing a circle, passive movements relating to gestures and gesture power may be indicative of the patient's ability to draw a circle. In this way, active performance scores may be calculated for particular tasks without the patient having to perform the task. Similarly, movement scores and/or performance scores calculated for a task based on the passive data and/or active performance data can be used to predict and/or analyze the performance of the patient on the task.

At step 218, a motor system assessment may be determined based upon the movement scores and/or performance scores. An impaired or not impaired motor system assessment may be determined using any of a variety of the passive/active movement data collection techniques as described herein.

At step 220, a treatment strategy may be determined. A motor system assessment may be generated based on the movement scores and/or performance scores. The motor system assessment may indicate if the patient's motor system is impaired or not impaired. A patient's passive movement score and active performance score may be compared to an established set of movement and performance scores from a particular patient-population. As discussed above, the threshold value, as determined by a particular patient-population may be used to establish a particular threshold that defines what particular movement or performance scores are defined as impaired or not impaired. For example, if the movement score is above a movement score threshold and the performance score is above a performance score threshold, as established and compared to the patient-population threshold value, the motor system assessment may indicate that the patient is not impaired. Similarly, if both the movement score and performance score are below the relevant thresholds, as established and compared to the patient-population threshold, the motor system assessment may indicate that the patient's movement is impaired. The impaired-ness of a patient may be a binary flag (*e.g.,* impaired or not impaired) and/or a graduated ranking of impaired-ness of the patient. For example, a patient that is above the movement score and/or performance score threshold may not be as impaired (and/or have a higher quality of life) than a patient that has a movement score and/or a performance score below the movement/performance score threshold irrespective of the patient's individual performance scores. By determining whether a patient's motor system assessment is impaired or not impaired, recommendations or commands for patient treatment based upon the severity of movement impairment may be determined. Such treatments may include therapeutically effective amounts of pharmaceuticals for the treatment of Parkinson's disease. Non-limiting examples of such treatments may include levodopa, sinemet, safinamide, carbidopa, dopamine agonists, COMT inhibitors, MAO-B inhibitors, amantadine, anticholinergics, and the like. In several embodiments, the treatment strategy may include physical therapy, tasks to be performed, and/or other activities to be performed by the patient.

At step 222, the determined treatment(s) may be administered. The treatment may be administered based on the determined treatment strategy. The provided treatment may include administering a therapeutically effective dose of one or more drugs as described herein. The therapeutically effective dose may be determined based on the passive movement scores and/or the active performance scores. For example, if the patient has an impaired quality of life due to hand tremors, a therapeutically effective dose of an appropriate drug may be administered to reduce the frequency of the hand tremors and thereby improve the patient's quality of life. In a second example, a patient having a low gesture strength may have particular physical movements administered in order to improve the patient's overall strength. As the patient's strength improves, the improvement in the patient's gesture power can be measured and tracked over time. As the patient continues to improve, more advanced physical movements may be administered to the patient to continue to improve the patient's motor system assessment and overall quality of life.

### EXAMPLE 1

The EQ-5D-5L is a self-assessed, health related, QoL questionnaire used to evaluate PD patients. The scale measures QoL on a 5-component scale including: 1) Mobility; 2) Self-Care; 3) Usual Activities; 4) Pain/Discomfort; and 5) Anxiety/Depression. Each level is rated on scale that describes the degree of problems in that area (i.e., no problem walking about, slight problem, moderate problem, severe problem, or unable to walk). The questionnaire also has an overall health scale where the PD patient selects a number between 1-100 to describe the condition of their health, 100 being the best imaginable. The EQ-5D-5L contains 3125 possible health states defined by combining one level from each scale, ranging from 11111 (full health) to 55555 (worst health).

As discussed above and according to one aspect disclosed herein, motor behavior of patients recently diagnosed with PD may be measured in daily life using sensors from a smartphone and/or wearable device such as a smart watch to assess PD patients using smart devices. Motor functioning in daily life is one of the most ecologically valid reflections of motor disease severity, but is difficult to quantify objectively. By monitoring motor behavior in patients recently diagnosed with PD, one can determine treatment protocols via smartphone- and smartwatch-based measurements of passively monitored motor behavior in daily life in these individuals. According to the systems and methods described herein, the motor behavior evaluation may correlate to the patient's EQ-5D-5L self-assessed health questionnaire.

As disclosed herein, a comprehensive digital biomarker smartphone application was used to collect and assess motor symptoms in de-novo diagnosed Parkinson patients. Movement data of the individuals was collected and a motor system assessment was generated that are associated with the patient's QoL survey data. The assessment accuracy and veracity was confirmed by cross-referencing data from the patient's EQ-5D-5L self-assessed health questionnaire.

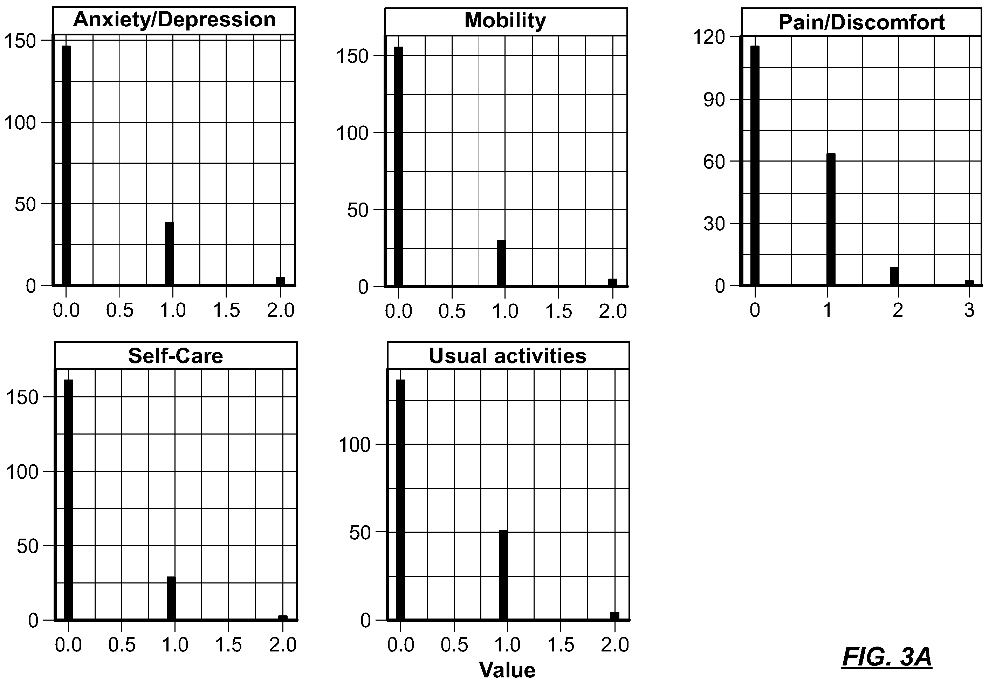

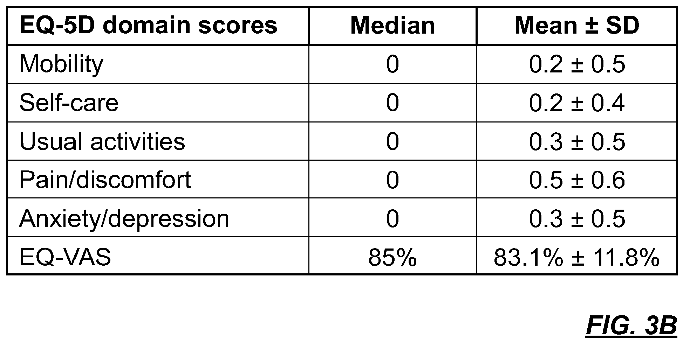

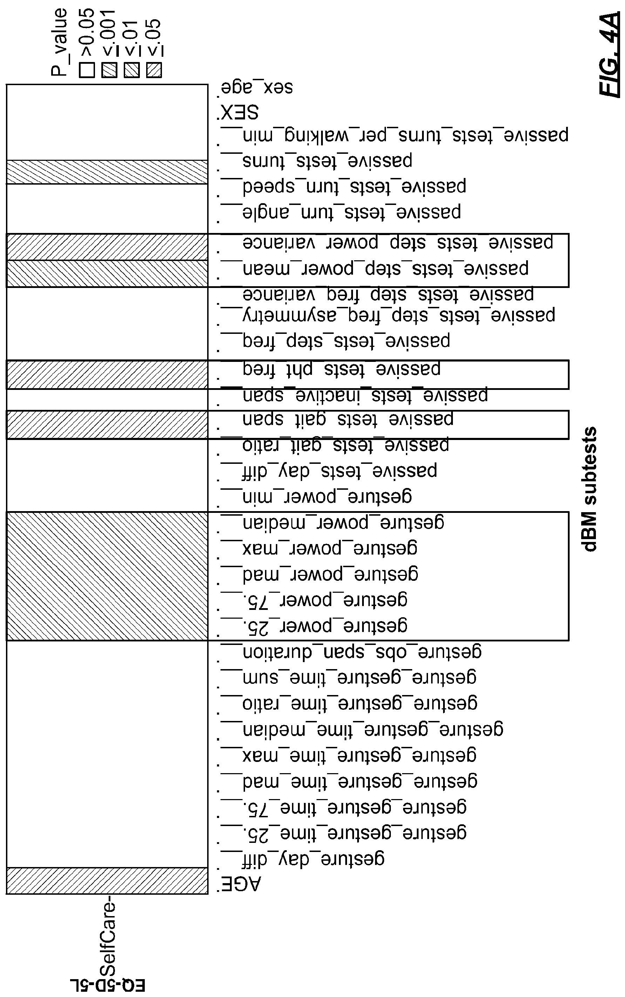

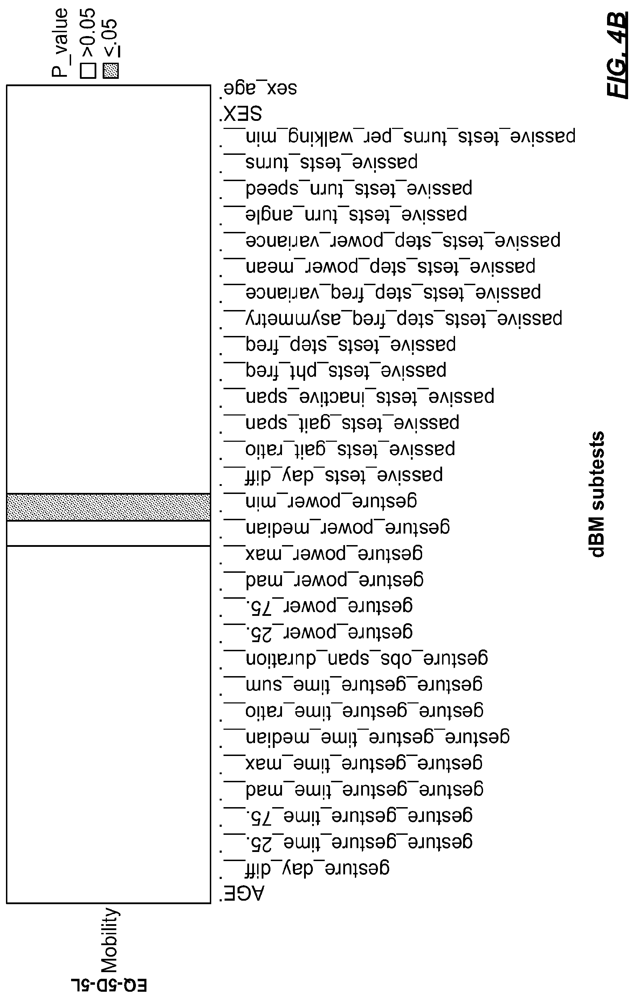

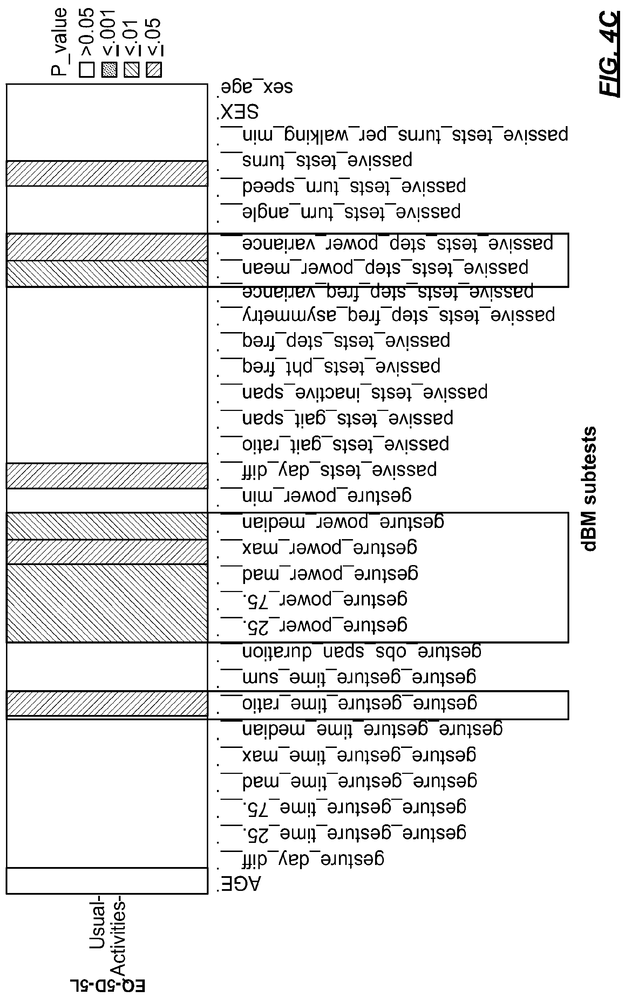

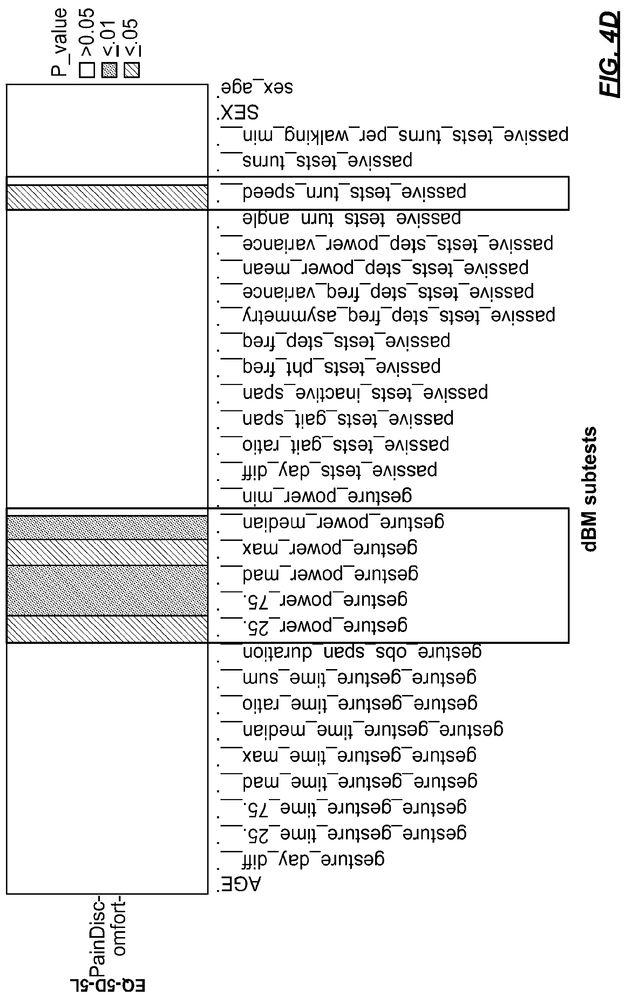

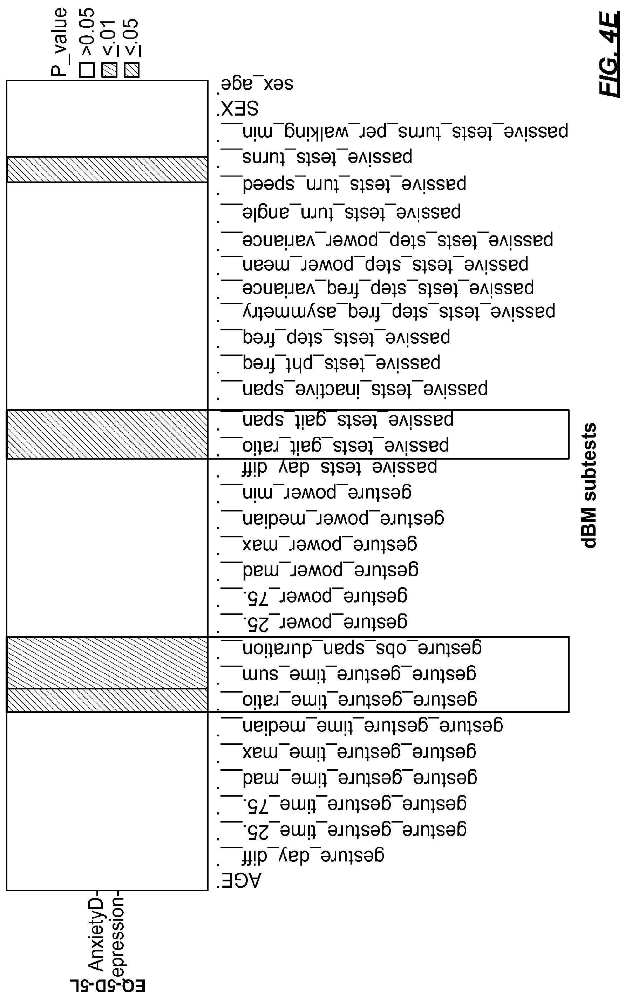

FIG. 3A graphically depicts the sample study distribution of the EQ-5D-5L domain scores across the five categories of Anxiety/Depression, Mobility, Pain/Discomfort, Self-Care, and Usual Activities. The study consisted of 191 individuals with a mean age of 59.9±9.2 years. PD diagnosis was previously confirmed by medical imaging. Individual EQ-5D-5L scores were grouped as impaired (scores > 0) and not-impaired (scores = 0). Logistic regression was conducted for EQ-5D-5L scores (EQ-5D-5L as dependent variable, residuals of sensor data, age, sex, age*sex as independent variables). Subsequently, patient movement data was collected by the mobile application disclosed herein via passive measures of gate using a smartphone and hand movement data collected via smartwatch, and averaged over two-week intervals. Sensor data were log-transformed and corrected for demographics (linear regression with age, sex, and the interaction of age/sex as covariates). Most impairment was shown in the Pain/Discomfort domain, Medium impairment in Usual Activities and Anxiety/Depression, and low impairment in Mobility and Self-Care. As depicted in FIGS. 4A-4E, passive movement data was collected across various designed categories to include, for example, gesture power, step power, gait span, pathological hand tremor (pht) frequency, gesture time, turn speed, and gesture span duration.

As depicted in FIGS. 4A-4E, test subject hand movement and gait feature data were significantly correlated to patients' judgements of difficulties with all EQ-5D-5L domains. In particular, the mobile application described herein determined, after collection and processing of patient passive movement data, that assessed movement impairment was shown to directly correlate with the patients' Pain/Discomfort domain, Medium impairment in Usual Activities and Anxiety/Depression, and low impairment in Mobility of the EQ-5D-5L self-assessed health questionnaire. Thus, the application disclosed herein may be used to generate a motor system assessment of a patient with PD in which gesture power is indicative of EQ-5D-5L Mobility data, in which gesture power, step power, gait span, and pathological hand tremor frequency are indicative of EQ-5D-5L Self-Care data, in which gesture power, step power, and gesture time data are indicative of EQ-5D-5L Usual Activities data, and in which gesture power and/or turn speed are indicative of EQ-5D-5L Pain/Discomfort data.

### EXAMPLE 2

Parkinson's Disease Questionnaire-39 (PDQ-39) is a self-assessed QoL questionnaire used to evaluate Parkinson's disease health related quality. The PDQ-39 is used to assess patient difficulty across eight main QoL dimensions, each with several sub-items, to include Mobility, Activities of Daily Living, Emotional Well-Being, Stigma, Social Support, Cognition, Communication, and Bodily Discomfort. The QoL dimensions may be scored on a five point ordinal system: 0 = Never, 1 = Occasionally, 2 = Sometimes, 3 = Often, 4 = Always.

Each PDQ-39 dimension total score may range from 0 (never have difficulty) to 100 (always have difficulty). The dimension score may be calculated by determining the sum of the sub-items in each dimension and multiplying by 100. Lower scores may reflect a better QoL. The overall PDQ-39 score may be calculated as the sum of the dimension scores divided by eight. The PDQ-39 questionnaire is used to assist health and social care professionals to determine the wider impact of Parkinson's disease on a person's quality of life, and be reevaluated to detect any changes following treatment for the disease.

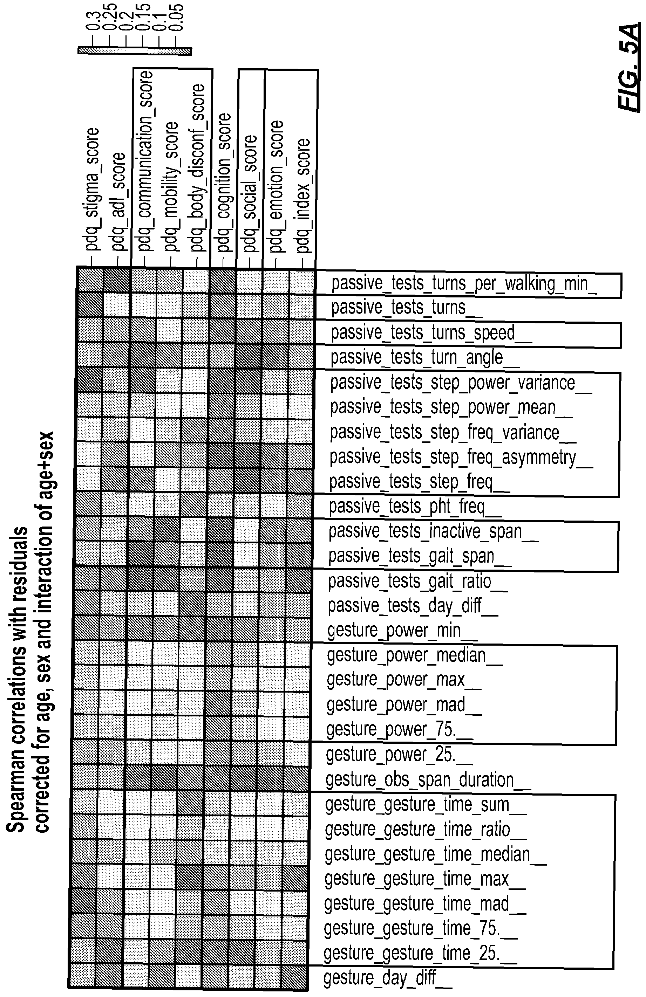

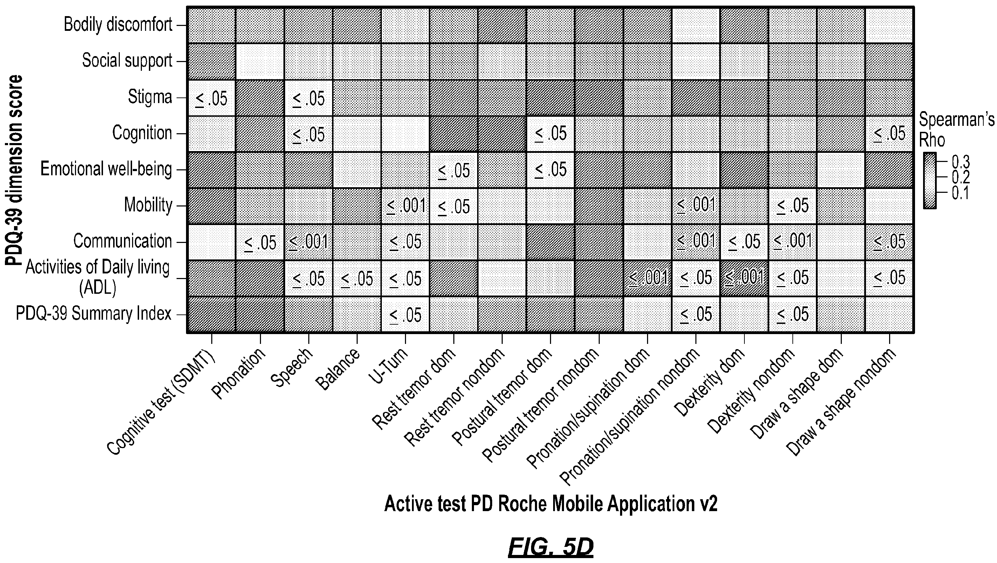

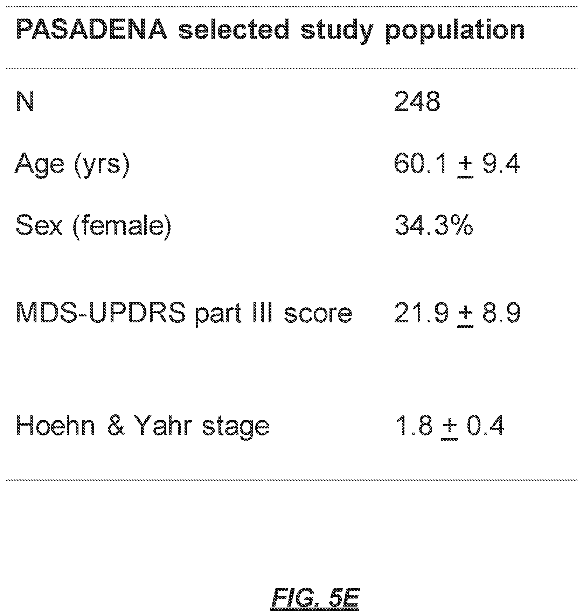

Passive movement data from 248 recently diagnosed individuals (less than two years) participating in a Phase II Study to Evaluate the Efficacy of Prasinezumab in Participants With Early Parkinson's Disease (PASADENA) were analyzed using the systems and methods disclosed herein. Passive measures of gait data collected via smartphone, and hand movement data collected via smartwatch were assessed using the mobile application disclosed herein. The mobile application collected the passive movement data and the data was averaged over two-week intervals. Collected sensor data were log transformed, and corrected for demographics. The association of sensor features and data analysis was compared to the subjects' PDQ-39 dimension scores using regression analyses and Spearman correlation. As shown in FIG. 5A, passive data collection includes gesture time, gesture power, gait, step, and turns. As also depicted in FIG. 5A, test subject passive hand movement features were significantly related to patients' judgements of difficulties with PDQ-39 Communication and Mobility dimensions. Analyzed passive gait data also significantly correlated to the patients' PDQ-39 Mobility and Bodily Discomfort dimensions.

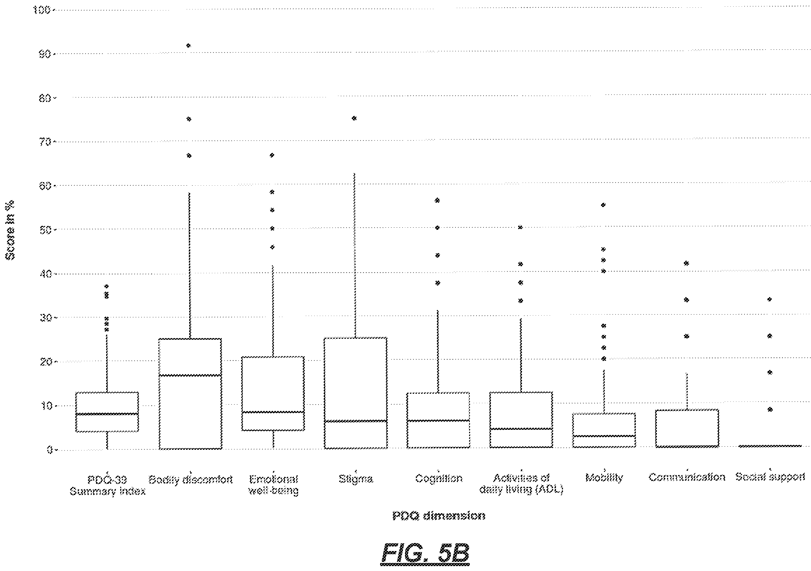

In addition, active movement data from the 248 PD individuals were collected via smartphone and/or smartwatch, and analyzed using the mobile application disclosed herein. Test subject distributions of PDQ-39 dimension scores at screening is graphically depicted in FIG. 5B in which higher scores indicate increased movement impairment. Test group subjects with PD endorsed few PDQ-39 impacts. As shown in FIG. 5B, overall QoL, as measured by the PDQ-39, was relatively high in the test population (*i.e.,* low PDQ-39 index and domain scores), resulting in truncation of ranges in some PDQ-39 dimension scores. For example, on the item level, 50% of individuals with PD reported no difficulties on the majority of items, especially items related to lower extremity mobility problems and lack of social support showed little endorsement. In this cohort, individuals with PD mainly reported bodily discomfort (*i.e.,* pain or cramps) and PD-related impact on emotional well-being and stigma ("having to hide it," "being worried about the future," "feeling anxious or depressed") impaired memory, problems concentrating difficulties with primarily upper-body related activities of daily living (*e.g.,* writing clearly, difficulties with buttons and shoelaces).

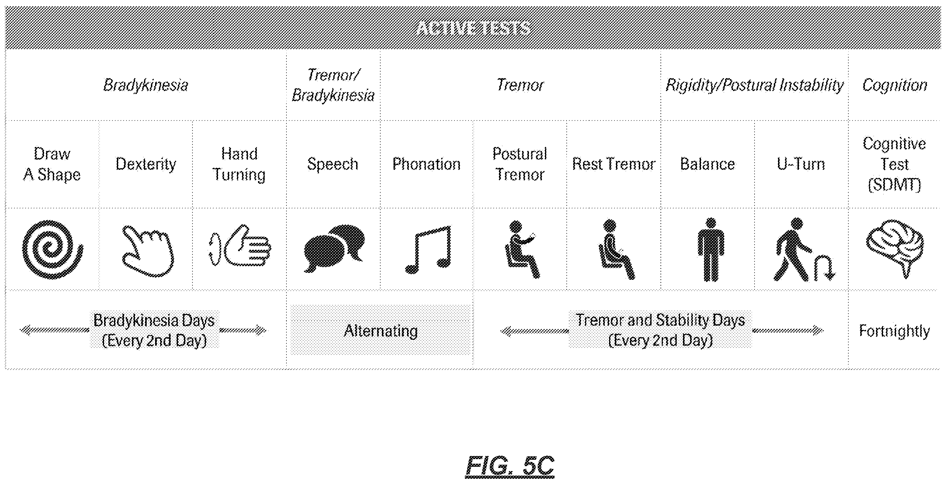

The mobile application includes active testing features to assess bradykinesia, tremor/bradykinesia, tremor, rigity/postural instability, and cognition. As shown in FIG. 5C, collected active movements include drawing a shape, dexterity, hand turning, speech, phonation, postural tremor, rest tremor, balance, U-turn, and cognitive Symbol Digit Modalities Test (SDMT). The active movement tests were administered to the test subjects using the mobile application disclosed herein. One *a priori* defined sensor-based feature per active test was extracted, averaged over a two-week interval, and subsequently compared with PDQ-39 dimension and index scores (Spearman correlation), and PDQ-39 items, classified as non-impaired (score = 0) vs. impaired (score ≥ 0) (Mann-Whitney U-test),

Active testing sensor features and collected data, surprisingly, correlated and were predictive of PDQ-39 scores. For example, as shown in FIG. 5C, various sensor features correlated to the test subjects' PDQ-39 dimensions including Activities of Daily Living, Communication, and Mobility. In particular, dominant hand dexterity and dominant hand pronation/supination features directly correlated and predicted test subject Activities of Daily Living PDQ-39 domains. *See* FIG. 5C. These active testing features were also correlated with and predictive of Communication and Mobility PDQ-39 domains. *See* FIG. 5C. A similar pattern of correlations and predictions was observed for the non-dominant hand draw a shape feature despite a decreased sample size (N=85) for this comparison. The PDQ-39 Communication domain was correlated and predicted by changes in a speech sensor feature of the application, and the PDQ-39 Mobility domain was correlated and predicted by the U-turn test sensor feature. *See* FIG. 5C. Emotional Well-Being, Cognition, and Stigma showed lower correlations with testing sensor features. Body Discomfort and Social Support did not significantly correlate with any of the pre-selected testing sensor features.

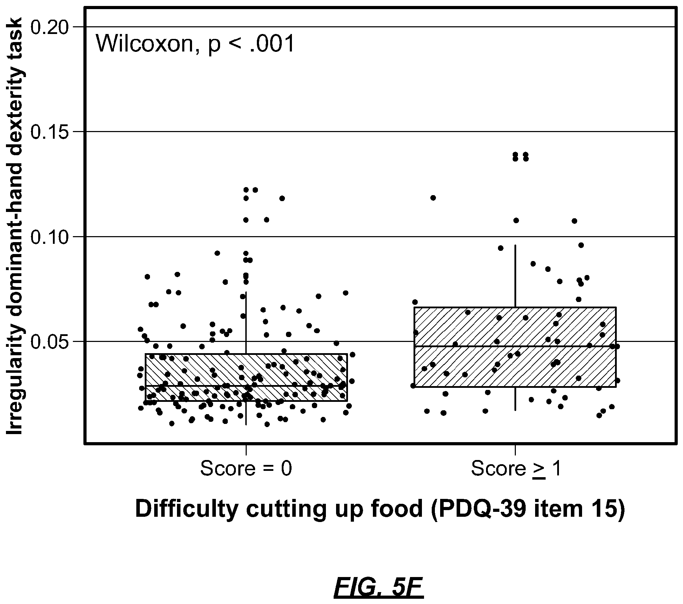

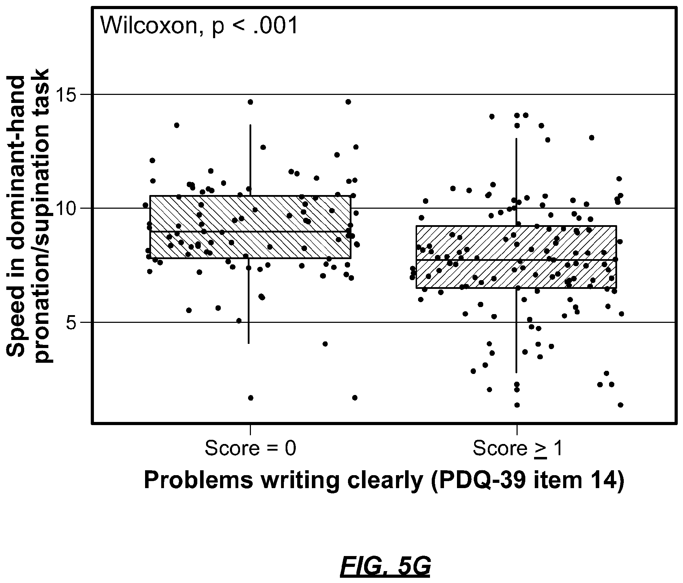

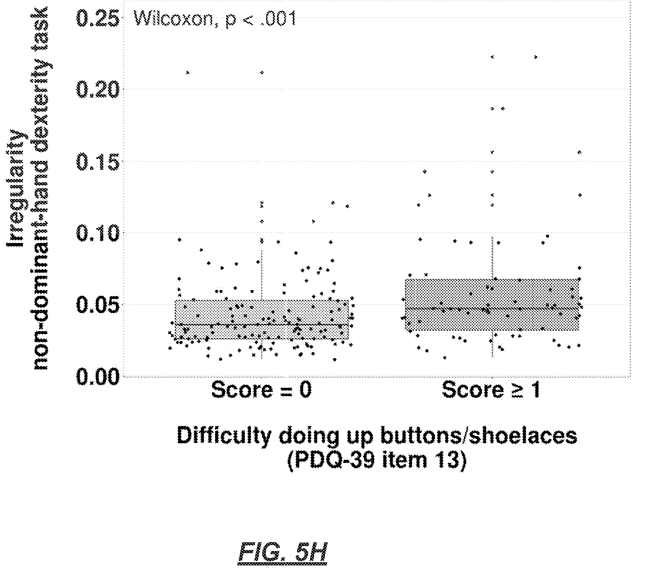

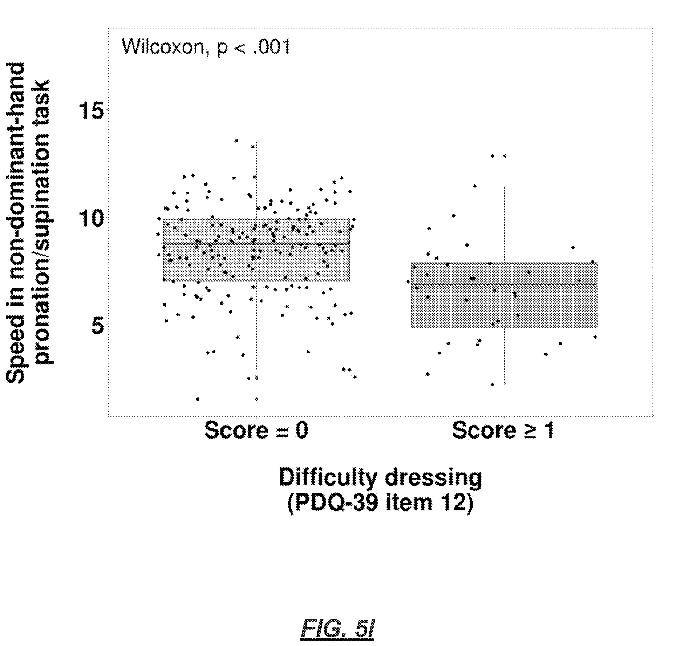

As shown in FIGS. 5F-5I, active testing features of the application also correlated to PDQ-39 item-level analyses. Difficulty getting around the house was associated with slower turning speed, and difficulties in Activities of Daily Living requiring hand movements were associated with irregularity in finger tapping and decreased hand-turning speed. Greater irregularity of finger tapping movements was observed in patients reporting troubles writing (54% increase in irregularity) or cutting food (59% increase in irregularity, both p < .001). Overall, difficulties with one-handed activities were associated with poorer dominant hand active test feature values, as shown in FIG. 5F and 5G, whereas difficulties with activities requiring both hands was associated with poorer performance in non-dominant hand active tests, as shown in FIGS. 5H and 5I.

The mobile application and related active testing/sensor features accurately determined subject motor symptom severity, as confirmed by the individual's PDQ-39 assessment. Importantly, the mobile application detected differences despite relatively low levels of test subject impairment, especially in the PDQ-39 domains of Activities of Daily Living, Communication, and Mobility. The findings disclosed herein suggest that the application analysis may be stronger in individuals with more advanced Parkinson's disease.

### EXAMPLE 3

The Movement Disorder Society-Unified Parkinson's Disease Rating Scale (MDS-UPDRS) is a comprehensive QoL assessment designed to monitor the severity of Parkinson's disease across the longitudinal disease course and provide clinical recommendations to healthcare providers. The MDS-UPDRS assessment includes 50 comprehensive questions to asses both motor and non-motor symptoms associated with PD. The MDS-UPDRS includes four parts with a total summed score. The components of the assessment include: Part I non-motor experiences of daily living (13 items), Part II motor experiences of daily living (13 items), Part III motor examination (18 items), and Part IV motor complications (six items). Part I includes two components, Part IA that concerns a number of behaviors assessed by an investigator with pertinent patient and caregiver information, and Part IB completed solely by the patient. Each subscale has a rating of 0-4, where 0 = normal, 1 = slight, 2 = mild, 3 = moderate, and 4 = severe. Higher scores indicate a greater severity and impact of Parkinson's disease symptoms.

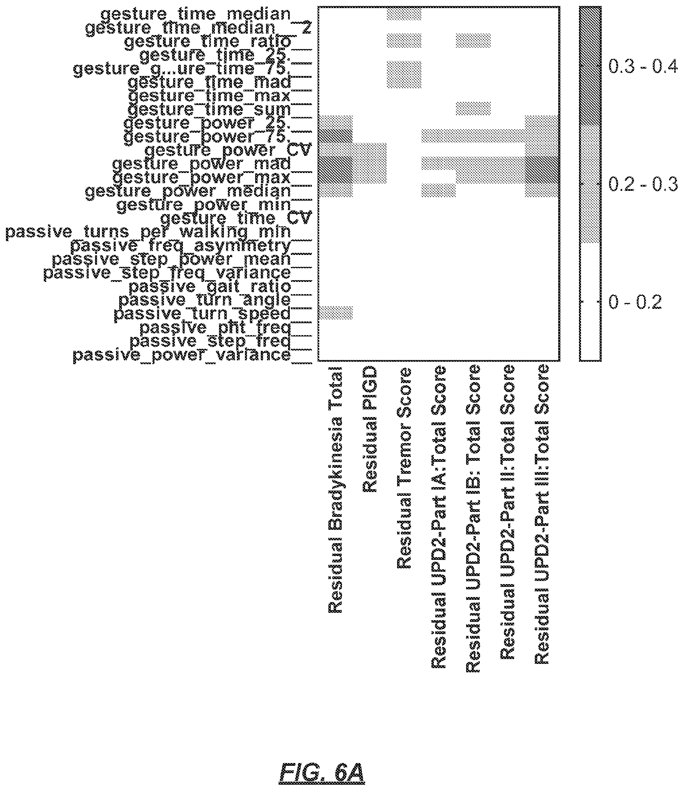

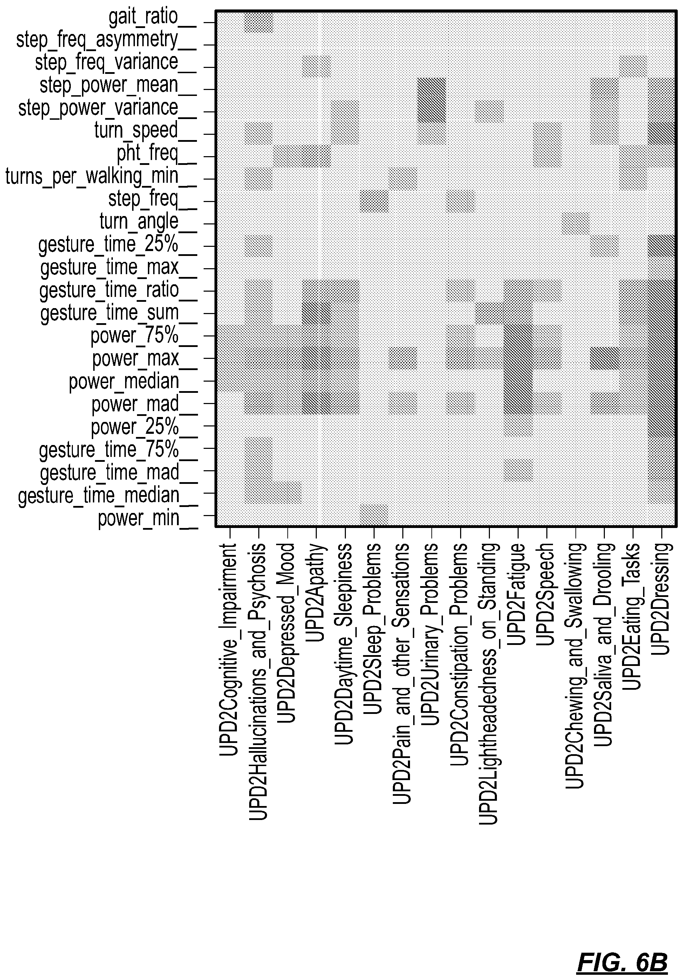

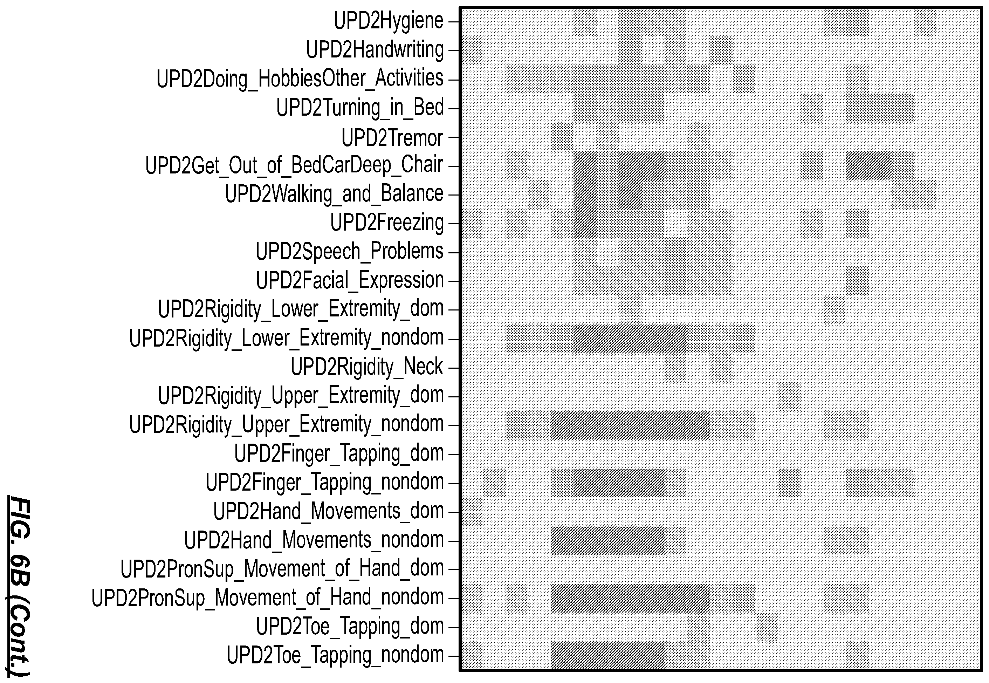

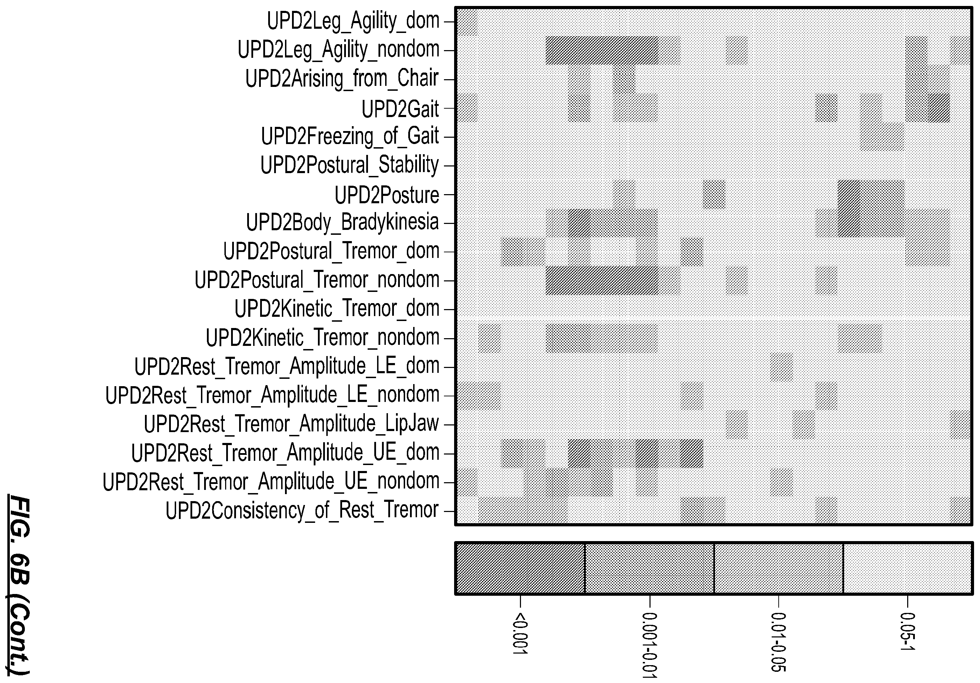

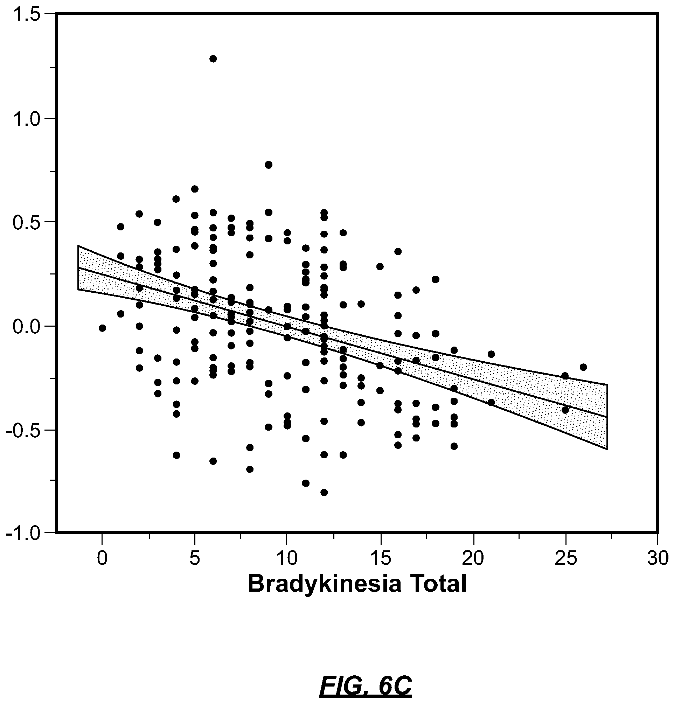

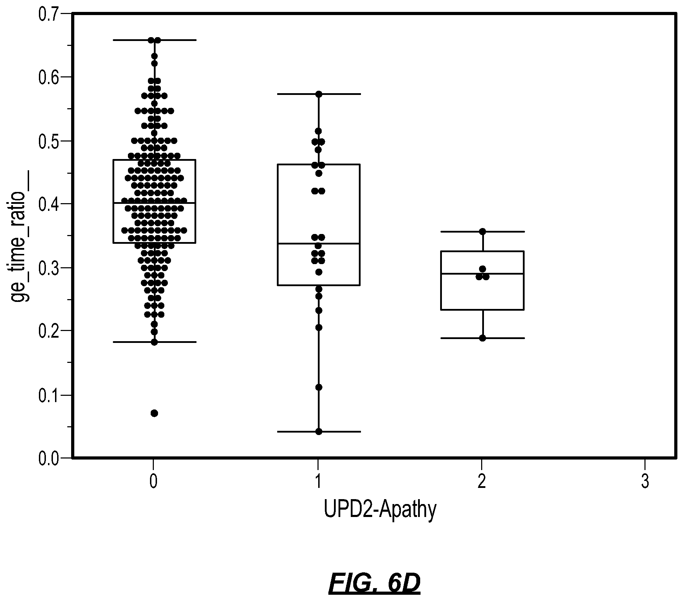

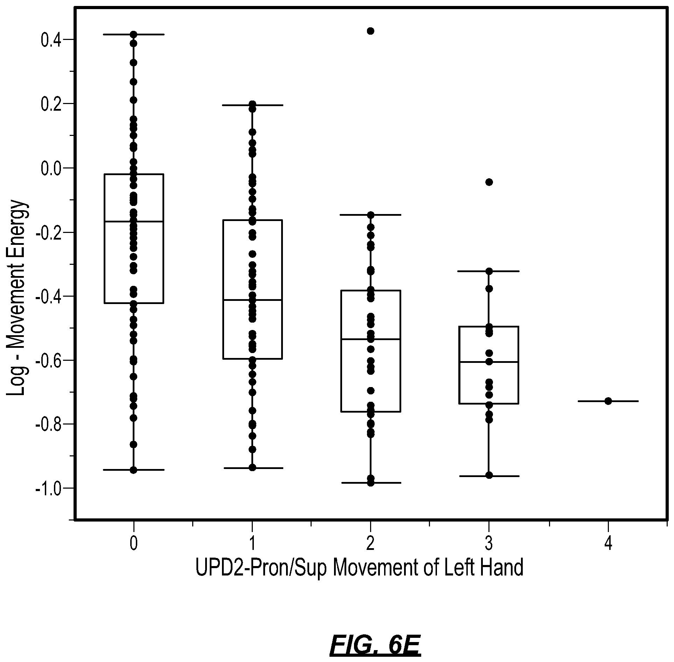

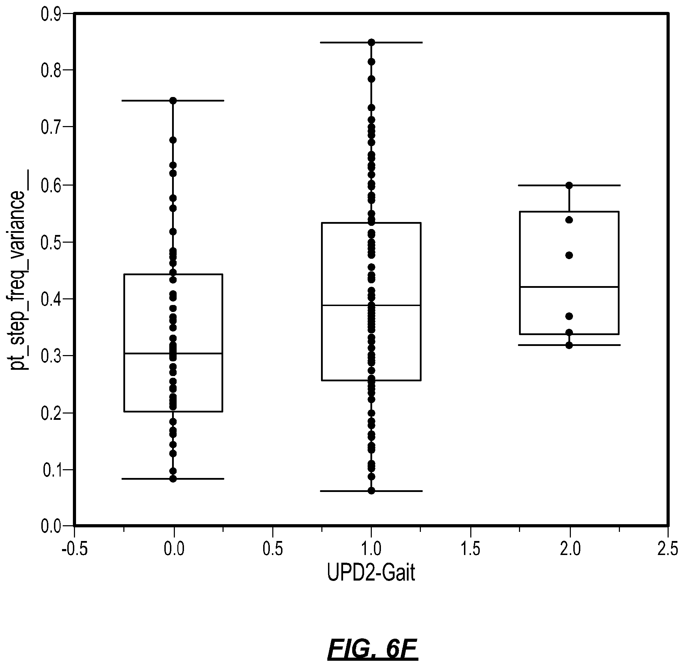

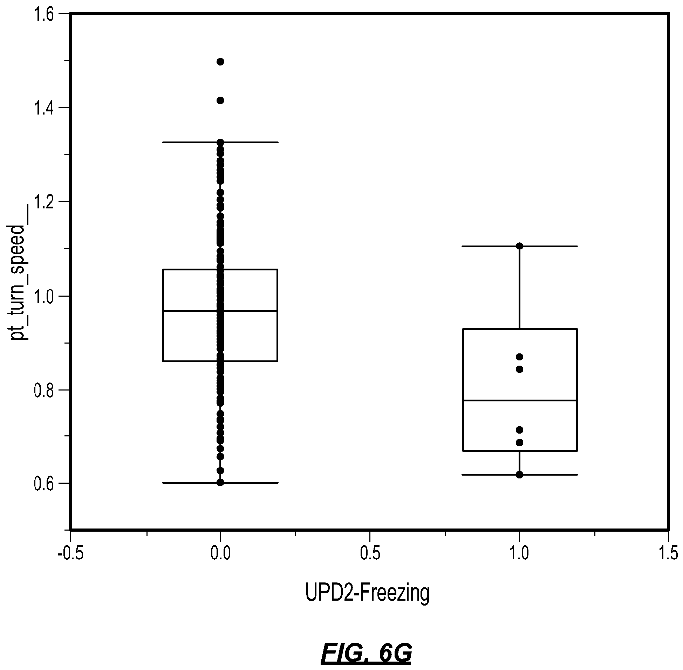

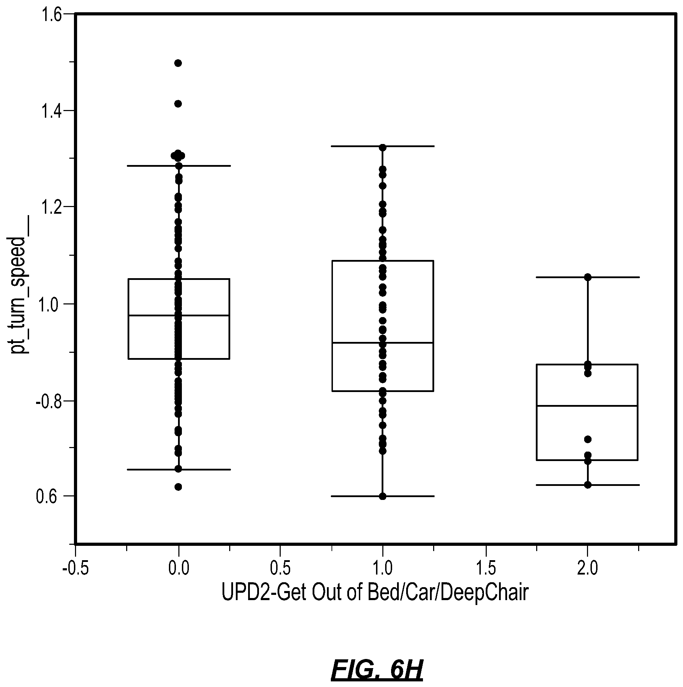

As discussed above, the passive movement data from 248 recently diagnosed individuals (less than two years) participating in a Phase II Study to Evaluate the Efficacy of Prasinezumab in Participants With Early Parkinson's Disease (PASADENA) were analyzed using the systems and methods disclosed herein. Data from active tests and continuous passive movement data were collected via smartphone and/or smartwatch using the mobile application disclosed herein. Collected sensor data were corrected for age and gender. The association of sensor features and data analysis was compared to the subjects' MDS-UPDRS item data scores using Spearman correlation for the composite scores. (Spearman correlations and Mann-Whitney tests (=0 "non-impaired," >0 "impaired"). As shown in FIG. 6A, passive data collection includes gesture time, gesture power, turns per walking, frequency asymmetry, step power, step frequency variance, gait ratio, turn angle, turn speed, pathological hand tremor (pht) frequency, step frequency and/or power variance. As also depicted in FIGS. 6A and 6B, collected passive data significantly correlated with MDS-UPDRS Part III scores and residual bradykinesia.

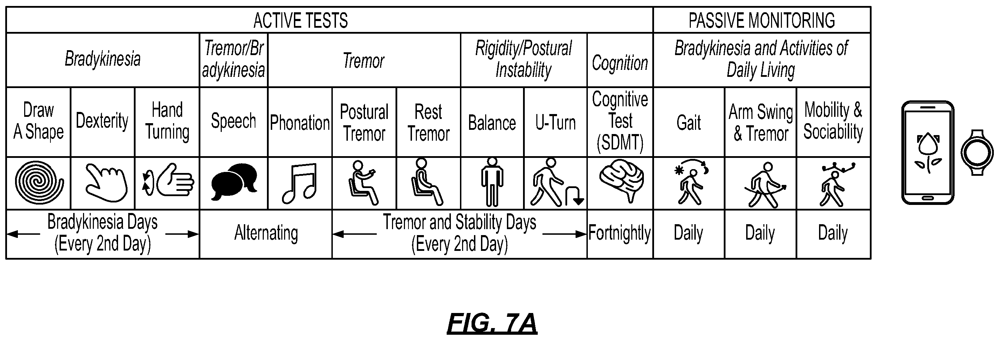

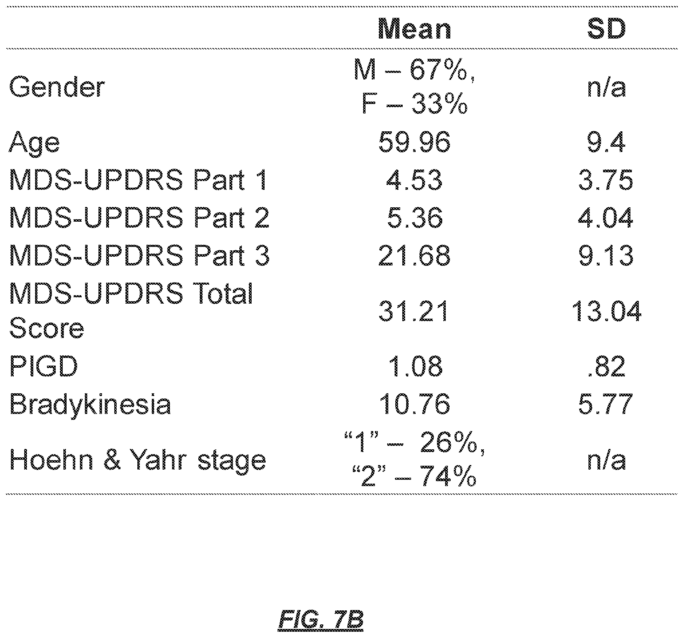

In addition, active and passive movement data from the 250 PD individuals were collected via smartphone and/or smartwatch, and analyzed using the mobile application disclosed herein. As illustrated in FIG. 7A, the mobile application includes active testing features to assess bradykinesia, tremor/bradykinesia, tremor, rigity/postural instability, and cognition, in addition to continuous passive monitoring of gait, arm swing/tremor, and mobility/sociability data. Collected active movements specifically includes drawing a shape, dexterity, hand turning, speech, phonation, postural tremor, rest tremor, balance, U-turn, and cognitive Symbol Digit Modalities Test (SDMT). Collected sensor data were corrected for age and gender, and test-retest reliability was assessed using intra-class correlation coefficients. Sensor data was averaged over +/- 7 days around the baseline visit. *See* FIG. 7B. Active and passive movement data was subsequently compared with MDS-UPDRS item and subscale scores by Spearman correlation and classified as non-impaired (score = 0) vs. impaired (score ≥ 0).

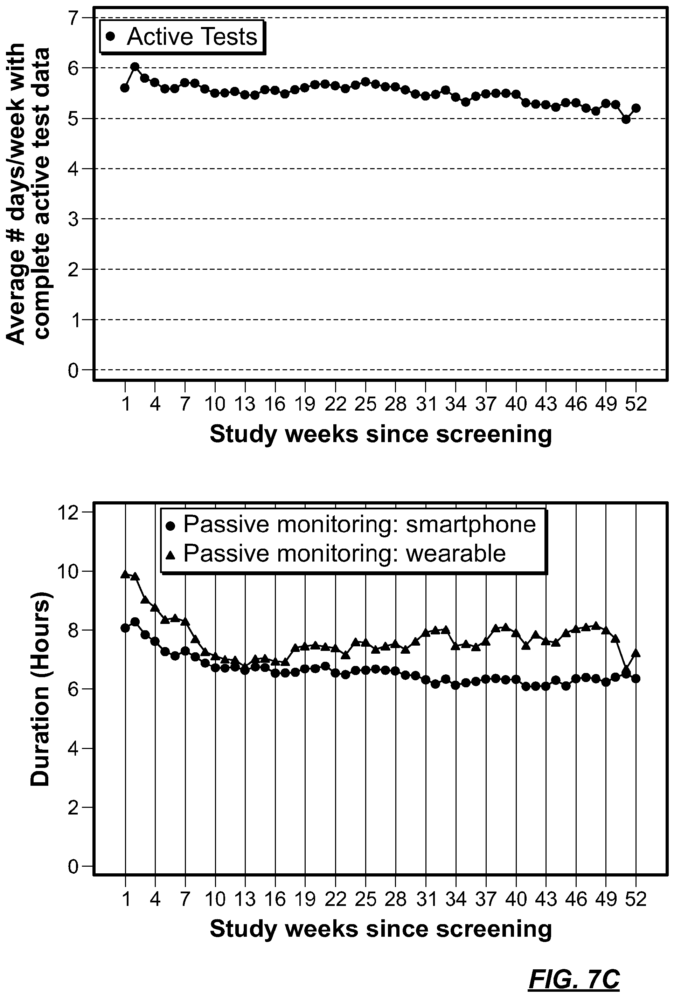

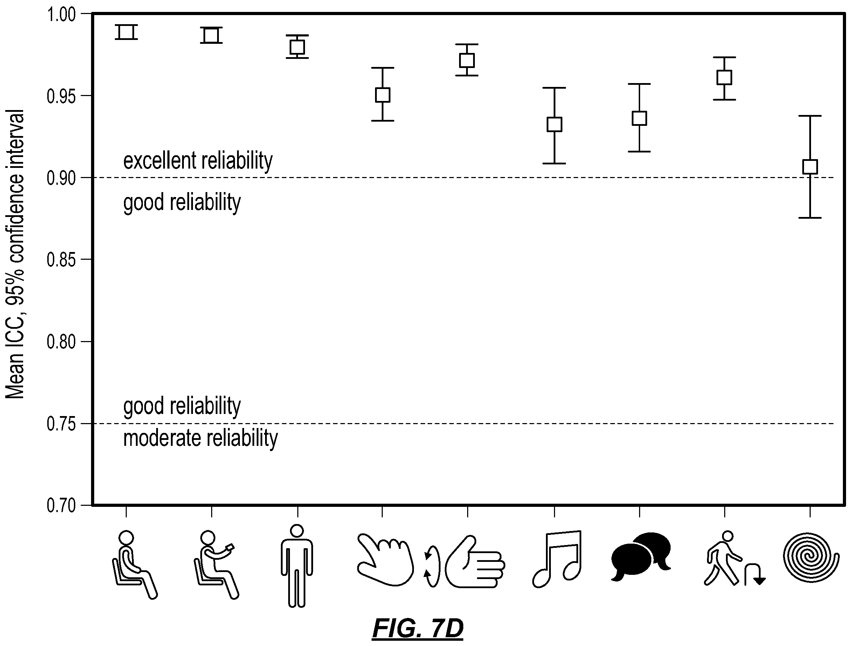

As shown in FIG. 7C, average adherence was high with 93% (median) of all possible active tests performed during the first two study weeks, and more than 7 hours of passive monitoring data collected daily. Test-retest reliability was assessed using intra-class correlation coefficients (ICCs). As shown in FIG. 7D, active test test-retest reliability (ICC's over two consecutive 14-day averages) was high (median 0.95, range 0.90-0.99).

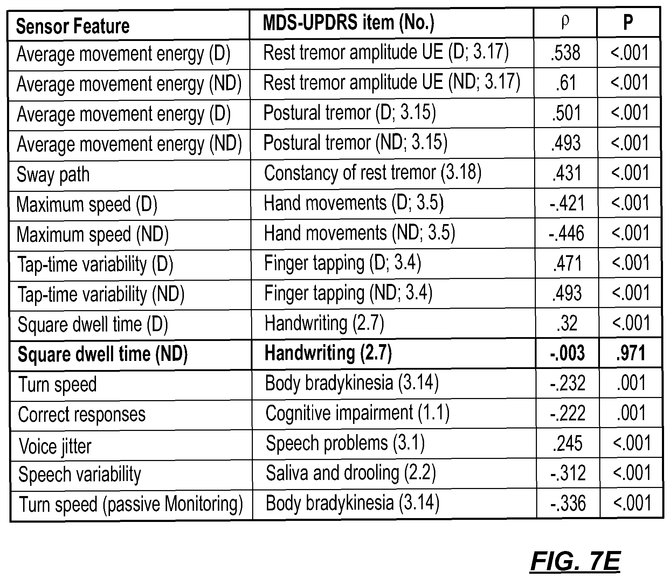

As depicted in FIG. 7E, active and passive testing sensor features and collected data, surprisingly, correlated and were predictive of MDS-UPDRS item and subscale scores. For example, average movement energy correlated to MDS-UPDRS rest tremor amplitude and postural tremor scores. Sway path (balance) correlated to MDS-UPDRS constancy of tremor score. Maximum speed correlated to MDS-UPDRS hand movement scores. Tap-time (dexterity) variability correlated to MDS-UPDRS finger tapping scores. Square dwell time (drawing a shape) correlated to MDS-UPDRS handwriting scores. Turn speed (U-turn) correlated to MDS-UPDRS body bradykinesia scores. Correct responses to SDMT correlated to MDS-UPDRS cognitive impairment scores. Voice jitter (phonation) correlated to MDS-UPDRS speech problem scores. Speech variability correlated to MDS-UPDRS saliva and drooling scores. And passive turn speed correlated to MDS-UPDRS body bradykinesia scores. In summary, all active tests significantly correlated with MDS-UPDRS item scores, ranging from r=-0.24 (p<.001; cognitive test) to r=.61 (p<.001; rest tremor). Average passive turn speed during daily life significantly correlated with MDS-UPDRS Part III (r=-.26, p<.001) and bradykinesia composite scores (r=-.34, p<.001).

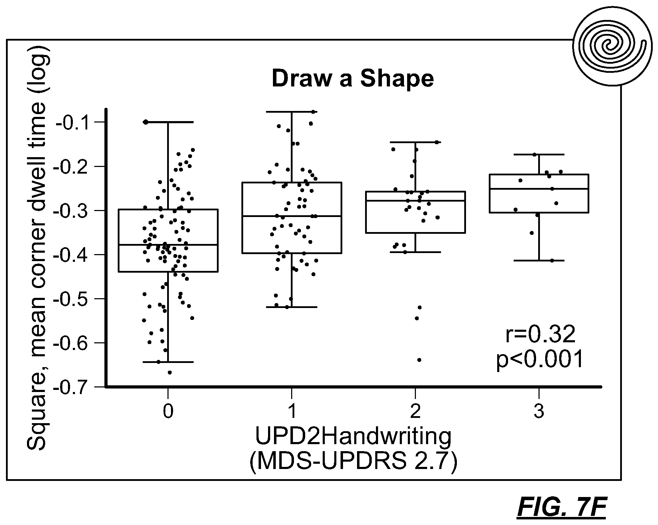

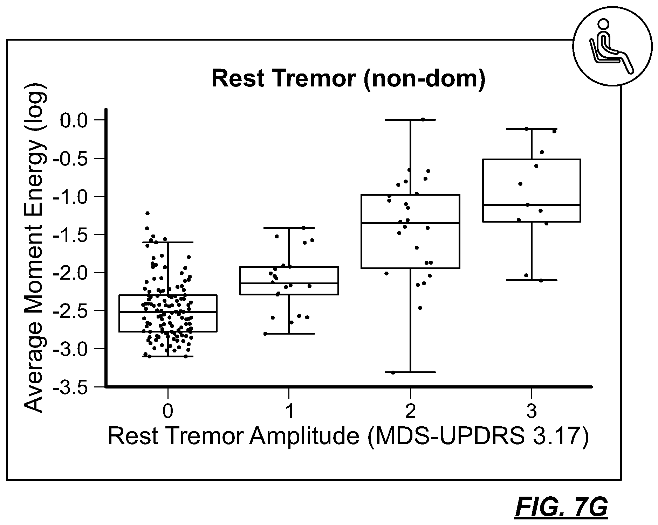

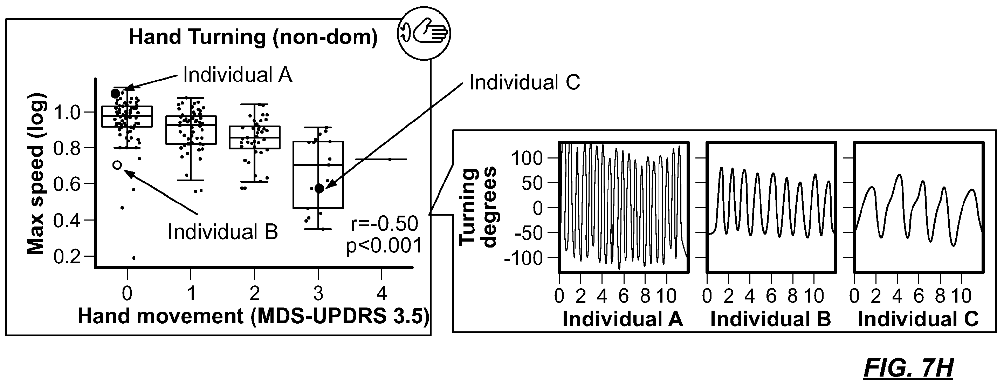

FIGS. 7F-7H further depict example relationships between sensor features and corresponding MDS-UPDRS item scores. For instance, as shown in FIGS. 7F and 7G, drawing a shape correlated to MDS-UPDRS handwriting scores, and rest tremor data correlated to MDS-UPDRS rest tremor amplitude scores. As shown in FIG. 7H, speed in hand turning correlated with scores in the MDS-UPDRS hand movement item. In addition, individuals showing no impairment on MDS-UPDRS displayed faster turning speed with higher turning amplitudes in the hand turning sensor feature (Individual A), compared to individuals with higher scores on MDS-UPDRS (Individual C). Individuals with no clinically apparent symptoms may still show significant impairment in hand-turning (Individual B).

The mobile application and related active testing/sensor features accurately determined subject motor symptom severity. The remote monitoring approach disclosed herein enables high-frequency sign and symptom assessment. Coupled with the high sensitivity of smartphone/-watch sensors, this may increase signal-to-noise in measurements of motor progression in clinical research and evaluation of a patient's health. The comprehensive measurement of PD core symptoms remotely, continuously, and objectively, according to the systems and methods described herein will provide unprecedented insight into PD patients' functioning.

By determining whether a patient's motor system assessment is impaired or not impaired, the system described herein may include additional recommendations or commands for patient treatment based upon the severity of movement impairment. Such treatments may include therapeutically effective amounts of pharmaceuticals for the treatment of Parkinson's disease. Non-limiting examples of such treatments may include levodopa, sinemet, safinamide, carbidopa, dopamine agonists, COMT inhibitors, MAO-B inhibitors, amantadine, anticholinergics, etc.

The passive and active movement data collected and processed by the novel mobile application disclosed herein validates the feasibility of remote digital monitoring of Parkinson's disease symptoms and will provide health professionals reliable and valid information about symptom severity, disease progression, and potential treatment protocols.

Although the present invention has been described in certain specific aspects, many additional modifications and variations would be apparent to those skilled in the art. In particular, any of the various processes described above may be performed in alternative sequences and/or in parallel (on different computing devices) in order to achieve similar results in a manner that is more appropriate to the requirements of a specific application. Thus, embodiments of the present invention should be considered in all respects as illustrative and not restrictive. Accordingly, the scope of the invention should be determined not by the embodiments illustrated, but by the appended claims and their equivalents.

## Claims

1. A computer-implemented method for assessing motor symptoms in Parkinson patients comprising:
obtaining (202), from a sensor, patient movement data wherein the movement data comprises passive movement data and active performance data, wherein the patient movement data comprises gesture power, step power, gait span, pathological hand tremor frequency, gesture time, turn speed, and gesture span duration;
determining that the patient movement data is impaired or not impaired; and
generating (204), based on the patient movement data, a motor system assessment, wherein the motor system assessment is impaired or not impaired.

2. The method of claim 1, wherein the sensor comprises a mobile telephone or a smart watch.

3. The method of claim 1, wherein the motor system assessment is highly correlative to EQ-5D-5L health status measurement data, and
wherein gesture power is indicative of EQ-5D-5L Mobility data;
wherein gesture power, step power, gait span, and pathological hand tremor frequency are indicative of EQ-5D-5L Self-Care data;
wherein gesture power, step power, and gesture time data are indicative of EQ-5D-5L Usual Activities data;
wherein gesture power and/or turn speed are indicative of EQ-5D-5L Pain/Discomfort data; and
wherein gesture time, gesture span duration, and gait span data are indicative of EQ-5D-5L Anxiety/Depression data.

4. The method of claim 1, wherein the patient active performance data comprises drawing a shape, dexterity, hand turning, speech, phonation, postural tremor, rest tremor, balance, U-turn, and cognitive test (Symbol Digit Modalities Test).

5. The method of claim 4, wherein the motor system assessment is highly correlative to quality of life (PDQ-39) measurements, and
wherein drawing a shape, dexterity, or hand turning is indicative of PDQ-39 Activities of Daily Living, Communication, or Mobility data;
wherein speech is indicative of PDQ-39 Communication data; and
wherein U-turn is indicative of PDQ-39 Mobility data.

6. The method of claim 1, wherein the patient active performance data comprises at least one of drawing a shape, dexterity, hand turning, speech, phonation, postural tremor, rest tremor, balance, U-turn, and cognitive test (Symbol Digit Modalities Test); and
wherein the patient passive movement data comprises at least one of gait, arm swing/tremor, and mobility/sociability.

7. The method of claim 6, wherein the motor system assessment is highly correlative to disease severity ratings (MDS-UPDRS), and
wherein rest tremor is indicative of MDS-UPDRS Rest Tremor Amplitude data;
wherein postural tremor is indicative of MDS-UPDRS Rest Tremor Amplitude data;
wherein balance is indicative of MDS-UPDRS Consistency of Rest Tremor data;
wherein hand turning is indicative of MDS-UPDRS Hand Movements data;
wherein dexterity is indicative of MDS-UPDRS Finger Tapping data;
wherein drawing a shape is indicative of MDS-UPDRS Handwriting data;
wherein U-turn is indicative of MDS-UPDRS Body Bradykinesia data;
wherein cognitive test (Symbol Digit Modalities Test) is indicative of MDS-UPDRS Cognitive Impairment data;
wherein phonation is indicative of MDS-UPDRS Speech Problems data;
wherein speech is indicative of MDS-UPDRS Saliva and Drooling data; and
wherein gait is indicative of MDS-UPDRS Body Bradykinesia data.

8. The method of claim 1, further comprising:
calculating (216), based on a patient-population movement data wherein the movement data comprises passive movement data and active performance data, a threshold value defining the motor system assessment as impaired or not impaired.

9. The method of claim 8, further comprising:
calculating (216) a patient movement and performance score,
comparing the patient movement and/or performance score to the threshold value,
and
determining (218) if the motor system assessment is impaired or not impaired.

10. The method of claim 9, further comprising:
displaying a command, wherein the command is no treatment for a not impaired motor system assessment; and
displaying a command, wherein the command is to initiate a treatment for an impaired motor system assessment.

11. The method of claim 10, further comprising:
calculating the treatment based on the motor system assessment, wherein the treatment is delivering a therapeutically effective amount of a drug to the patient.

12. A system for assessing motor symptoms in Parkinson patients comprising:
a display (119);
a sensor;
a processor (111); and
a memory (121), wherein the memory is in communication with the processor storing instructions that, when executed by the processor, cause the system to:
obtain, from the sensor, patient movement data wherein the movement data comprises passive movement data and active performance data wherein the patient passive movement data comprises gesture power, step power, gait span, pathological hand tremor frequency, gesture time, turn speed, and gesture span duration;
determine that the patient movement data is impaired or not impaired; and
generate, based on the patient movement data, a motor system assessment, wherein the motor system assessment is impaired or not impaired.

13. The system of claim 12, wherein the motor system assessment is highly correlative to EQ-5D-5L health status measurement data, and wherein gesture power is indicative of EQ-5D-5L Mobility data;
wherein gesture power, step power, gait span, and pathological hand tremor frequency are indicative of EQ-5D-5L Self-Care data;
wherein gesture power, step power, and gesture time data are indicative of EQ-5D-5L Usual Activities data;
wherein gesture power and/or turn speed are indicative of EQ-5D-5L Pain/Discomfort data; and
wherein gesture time, gesture span duration, and gait span data are indicative of EQ- 5D-5L Anxiety/Depression data.

14. The system of claim 12, wherein the instructions, when read by the processor, cause the system to further comprise:
calculate, based on a patient-population movement data wherein the movement data comprises passive movement data and/or active performance data, a threshold value defining the motor system assessment as impaired or not impaired;
calculate a patient movement/performance score;
compare the patient movement/performance score to the threshold value;
determine if the motor system assessment is impaired or not impaired;
display a command, wherein the command is no treatment for a not impaired motor system assessment;
display a command, wherein the command is to initiate a treatment for an impaired motor system assessment; and
calculate the treatment based on the motor system assessment, wherein the treatment is delivering a therapeutically effective amount of a drag to the Parkinson's disease patient.

## Patentansprüche

1. Computerimplementiertes Verfahren zum Beurteilen motorischer Symptome bei Parkinson-Patienten, umfassend:
Erhalten (202), von einem Sensor, von Bewegungsdaten des Patienten, wobei die Bewegungsdaten passive Bewegungsdaten und aktive Leistungsdaten umfassen, wobei die Bewegungsdaten des Patienten Gestenkraft, Schrittkraft, Gangspanne, Frequenz des pathologischen Handtremors, Gestenzeit, Wendeschwindigkeit und Dauer der Gestenspanne umfassen;
Bestimmen, dass die Bewegungsdaten des Patienten beeinträchtigt oder nicht beeinträchtigt sind; und
Erzeugen (204), auf Grundlage der Bewegungsdaten des Patienten, einer Beurteilung des motorischen Systems, wobei die Beurteilung des motorischen Systems beeinträchtigt oder nicht beeinträchtigt ist.

2. Verfahren nach Anspruch 1, wobei der Sensor ein Mobiltelefon oder eine Smartwatch umfasst.

3. Verfahren nach Anspruch 1, wobei die Beurteilung des motorischen Systems hochgradig mit den EQ-5D-5L-Daten einer Gesundheitsstatusmessung korreliert, und
wobei die Gestenkraft für die EQ-5D-5L-Daten zur Mobilität indikativ ist;
wobei die Gestenkraft, Schrittkraft, Gangspanne und Frequenz des pathologischen Handtremors für die EQ-5D-5L-Daten zur Selbstpflege indikativ sind;
wobei die Daten zur Gestenkraft, Schrittkraft und Gestenzeit für die EQ-5D-5L-Daten zu den üblichen Aktivitäten indikativ sind;
wobei die Gestenkraft und/oder Wendegeschwindigkeit für die EQ-5D-5L-Daten zu Schmerzen/Beschwerden indikativ sind; und
wobei die Daten zur Gestenzeit, Dauer der Gestenspanne und Gangspanne für die EQ-5D-5L-Daten zu Ängstlichkeit/Depression indikativ sind.

4. Verfahren nach Anspruch 1, wobei die aktiven Leistungsdaten des Patienten Zeichnen einer Gestalt, Geschicklichkeit, Wenden der Hand, Sprechen, Lautbildung, Haltungstremor, Ruhetremor, Gleichgewicht, Kehrtwendung und einen kognitiven Test (Symbol Digit Modalities Test) umfassen.

5. Verfahren nach Anspruch 4, wobei die Beurteilung des motorischen Systems hochgradig mit den Messungen der Lebensqualität (PDQ-39) korreliert, und
wobei das Zeichnen einer Gestalt, Geschicklichkeit oder Wenden der Hand für die PDQ-39-Daten zu Aktivitäten des täglichen Lebens, Kommunikation oder Mobilität indikativ ist;
wobei Sprechen für die PDQ-39-Daten zur Kommunikation indikativ ist; und
wobei Kehrtwendung für die PDQ-39-Daten zur Mobilität indikativ ist.

6. Verfahren nach Anspruch 1, wobei die aktiven Leistungsdaten des Patienten mindestens eines umfassen von Zeichnen einer Gestalt, Geschicklichkeit, Wenden der Hand, Sprechen, Lautbildung, Haltungstremor, Ruhetremor, Gleichgewicht, Kehrtwendung und kognitivem Test (Symbol Digit Modalities Test); und
wobei die passiven Bewegungsdaten des Patienten mindestens eines umfassen von Gang, Armschwung/-tremor und Mobilität/Soziabilität.

7. Verfahren nach Anspruch 6, wobei die Beurteilung des motorischen Systems hochgradig mit den Schweregradbewertungen der Erkrankung (MDS-UPDRS) korreliert, und
wobei der Ruhetremor für die MDS-UPDRS-Daten zur Ruhetremoramplitude indikativ ist;
wobei der Haltungstremor für die MDS-UPDRS-Daten zur Ruhetremoramplitude indikativ ist;
wobei das Gleichgewicht für die MDS-UPDRS-Daten zur Konsistenz des Ruhetremors indikativ ist;
wobei das Wenden der Hand für die MDS-UPDRS-Daten zur Handbewegung indikativ ist;
wobei die Geschicklichkeit für die MDS-UPDRS-Daten zum Fingertippen indikativ ist;
wobei das Zeichnen einer Gestalt für die MDS-UPDRS-Daten zur Handschrift indikativ ist;
wobei die Kehrtwendung für die MDS-UPDRS-Daten zur Körperbradykinesie indikativ ist;
wobei der kognitive Test (Symbol Digit Modalities Test) für die MDS-UPDRS-Daten zur kognitiven Beeinträchtigung indikativ ist;
wobei die Lautbildung für die MDS-UPDRS-Daten zu Problemen beim Sprechen indikativ ist;
wobei das Sprechen für die MDS-UPDRS-Daten zu Speichel und Hypersalivation indikativ ist; und
wobei der Gang für die MDS-UPDRS-Daten zu Körperbradykinesie indikativ ist.

8. Verfahren nach Anspruch 1, weiter umfassend:
Berechnen (216), auf Grundlage von Bewegungsdaten einer Patientenpopulation, wobei die Bewegungsdaten passive Bewegungsdaten und aktive Leistungsdaten umfassen, eines Schwellenwerts, der die Beurteilung des motorischen Systems als beeinträchtigt oder nicht beeinträchtigt definiert.

9. Verfahren nach Anspruch 8, weiter umfassend:
Berechnen (216) einer Bewegungs- und Leistungspunktzahl des Patienten,
Vergleichen der Bewegungs- und/oder Leistungspunktzahl des Patienten mit dem Schwellenwert und
Bestimmen (218), ob die Beurteilung des motorischen Systems beeinträchtigt oder nicht beeinträchtigt ist.

10. Verfahren nach Anspruch 9, weiter umfassend:
Anzeigen eines Befehls, wobei der Befehl bei einer Beurteilung eines nicht beeinträchtigten motorischen Systems keine Behandlung ist; und
Anzeigen eines Befehls, wobei der Befehl bei einer Beurteilung eines beeinträchtigten motorischen Systems ist, eine Behandlung einzuleiten.

11. Verfahren nach Anspruch 10, weiter umfassend:
Berechnen der Behandlung auf Grundlage der Beurteilung des motorischen Systems, wobei die Behandlung das Abgeben einer therapeutisch wirksamen Menge eines Medikaments an den Patienten ist.

12. System zum Beurteilen motorischer Symptome bei Parkinson-Patienten, umfassend:
eine Anzeige (119);
einen Sensor;
einen Prozessor (111); und
einen Speicher (121), wobei der Speicher in Kommunikation mit dem Prozessor, der Anweisungen speichert, vorliegt, die, wenn von dem Prozessor ausgeführt, das System veranlassen zum:
Erhalten, von dem Sensor, von Bewegungsdaten des Patienten, wobei die Bewegungsdaten passive Bewegungsdaten und aktive Leistungsdaten umfassen, wobei die passiven Bewegungsdaten des Patienten Gestenkraft, Schrittkraft, Gangspanne, Frequenz des pathologischen Handtremors, Gestenzeit, Wendeschwindigkeit und Dauer der Gestenspanne umfassen;
Bestimmen, dass die Bewegungsdaten des Patienten beeinträchtigt oder nicht beeinträchtigt sind; und
Erzeugen, auf Grundlage der Bewegungsdaten des Patienten, einer Beurteilung des motorischen Systems, wobei die Beurteilung des motorischen Systems beeinträchtigt oder nicht beeinträchtigt ist.

13. System nach Anspruch 12, wobei die Beurteilung des motorischen Systems hochgradig mit den EQ-5D-5L-Daten zur Gesundheitszustandsmessung korreliert und die Gestenkraft für die EQ-5D-5L-Daten zur Mobilität indikativ ist;
wobei die Gestenkraft, Schrittkraft, Gangspanne und Frequenz des pathologischen Handtremors für die EQ-5D-5L-Daten zur Selbstpflege indikativ sind;
wobei die Daten zur Gestenkraft, Schrittkraft und Gestenzeit für die EQ-5D-5L-Daten zu den üblichen Aktivitäten indikativ sind;
wobei die Gestenkraft und/oder Wendegeschwindigkeit für die EQ-5D-5L-Daten zu Schmerzen/Beschwerden indikativ sind; und
wobei die Daten zur Gestenzeit, Dauer der Gestenspanne und Gangspanne für die EQ-5D-5L-Daten zu Ängstlichkeit/Depression indikativ sind.

14. System nach Anspruch 12, wobei die Anweisungen, wenn von dem Prozessor gelesen, das System veranlassen, weiter zu umfassen:
Berechnen, auf Grundlage von Bewegungsdaten einer Patientenpopulation, wobei die Bewegungsdaten passive Bewegungsdaten und/oder aktive Leistungsdaten umfassen, eines Schwellenwerts, der die Beurteilung des motorischen Systems als beeinträchtigt oder nicht beeinträchtigt definiert;
Berechnen einer Bewegungs-/Leistungspunktzahl des Patienten;
Vergleichen der Bewegungs-/Leistungspunktzahl des Patienten mit dem Schwellenwert;
Bestimmen, ob die Beurteilung des motorischen Systems beeinträchtigt oder nicht beeinträchtigt ist;
Anzeigen eines Befehls, wobei der Befehl bei einer Beurteilung eines nicht beeinträchtigten motorischen Systems keine Behandlung ist;
Anzeigen eines Befehls, wobei der Befehl bei einer Beurteilung eines beeinträchtigten motorischen Systems ist, eine Behandlung einzuleiten; und
Berechnen der Behandlung auf Grundlage der Beurteilung des motorischen Systems, wobei die Behandlung Abgeben einer therapeutisch wirksamen Menge eines Medikaments an den Patienten mit Parkinson-Erkrankung ist.

## Revendications

1. Procédé informatique d'évaluation des symptômes moteurs chez les patients atteints de la maladie de Parkinson comprenant :
l'obtention (202), à partir d'un capteur, de données de mouvement du patient dans lequel les données de mouvement comprennent des données de mouvement passif et des données de performance active, dans lequel les données de mouvement du patient comprennent la puissance du geste, la puissance du pas, l'étendue de la marche, la fréquence du tremblement pathologique de la main, le temps du geste, la vitesse de rotation et la durée de l'étendue du geste ;
la détermination si les données relatives aux mouvements du patient sont altérées ou non ; et
la génération (204), sur la base des données de mouvement du patient, d'une évaluation du système moteur, dans lequel l'évaluation du système moteur est altérée ou non.

2. Procédé selon la revendication 1, dans lequel le capteur comprend un téléphone mobile ou une montre intelligente.

3. Procédé selon la revendication 1, dans lequel l'évaluation du système moteur est fortement corrélée aux données de mesure de l'état de santé EQ-5D-5L, et
dans lequel la puissance du geste est indicative des données de mobilité EQ-5D-5L ;
dans lequel la puissance du geste, la puissance du pas, l'étendue de la marche et la fréquence des tremblements pathologiques de la main sont indicatives des données d'auto-soins EQ-5D-5L ;
dans lequel les données relatives à la puissance du geste, à la puissance du pas et au temps de geste sont indicatives des données relatives aux activités habituelles de l'EQ-5D-5L ;
dans lequel la puissance de geste et/ou la vitesse de rotation indiquées correspondent à des données douleur/inconfort de l'EQ-5D-5L ; et
dans lequel le temps de geste, la durée de l'étendue du geste et les données d'étendue de la marche sont indicatives des données d'anxiété/de dépression de l'EQ-5D-5L.

4. Procédé selon la revendication 1, dans lequel les données de performance active du patient comprennent le dessin d'une forme, la dextérité, la rotation de la main, la parole, la phonation, le tremblement postural, le tremblement au repos, l'équilibre, le demi-tour et le test cognitif (test des modalités de symboles et de chiffres).

5. Procédé selon la revendication 4, dans lequel l'évaluation du système moteur est fortement corrélée aux mesures de la qualité de vie (PDQ-39), et
dans lequel le dessin d'une forme, la dextérité ou la rotation de la main sont indicatifs des données d'activités de la vie quotidienne, communication ou mobilité PDQ-39 ;
dans lequel la parole est indicative des données de communication PDQ-39 ; et
dans lequel le demi-tour est indicatif des données de mobilité PDQ-39.

6. Procédé selon la revendication 1, dans lequel les données relatives aux performances actives du patient comprennent au moins un parmi : le dessin d'une forme, la dextérité, la rotation de la main, la parole, la phonation, le tremblement postural, le tremblement au repos, l'équilibre, le demi-tour et le test cognitif (test des modalités de symboles et de chiffres) ; et
dans lequel les données sur les mouvements passifs du patient comprennent au moins un élément parmi la marche, le balancement/tremblement du bras, et mobilité/sociabilité.

7. Procédé selon la revendication 6, dans lequel l'évaluation du système moteur est fortement corrélée aux scores de gravité de la maladie (MDS-UPDRS), et
dans lequel le tremblement au repos est indicatif des données d'amplitude du tremblement au repos MDS-UPDRS ;
dans lequel le tremblement postural est indicatif des données d'amplitude du tremblement au repos MDS-UPDRS ;
dans lequel l'équilibre est indicatif de la cohérence des données de tremblement au repos MDS-UPDRS ;
dans lequel la rotation de la main est indicative des données de mouvements de la main MDS-UPDRS ;
dans lequel la dextérité est indicative des données de tapotement des doigts MDS-UPDRS ;
dans lequel le dessin d'une forme est indicatif des données d'écriture manuscrite MDS-UPDRS ;
dans lequel le demi-tour est indicatif des données de bradykinésie corporelle MDS-UPDRS ;
dans lequel le test cognitif (test de modalités de symboles et de chiffres) est indicatif des données sur la déficience cognitive MDS-UPDRS ;
dans lequel la phonation est indicative des données relatives aux problèmes de parole MDS-UPDRS ;
dans lequel la parole est indicative des données de salive et de bave MDS-UPDRS ; et
dans lequel la démarche est indicative des données de bradykinésie corporelle MDS-UPDRS.

8. Procédé selon la revendication 1, comprenant en outre :
le calcul (216), sur la base des données de mouvement de population de patients dans lequel les données de mouvement comprennent des données de mouvement passif et des données de performance active, d'une valeur seuil définissant l'évaluation du système moteur comme étant altérée ou non.

9. Procédé selon la revendication 8, comprenant en outre :
le calcul (216) d'un score de mouvement et de performance du patient,
la comparaison des mouvements du patient et/ou du score de performance à la valeur seuil, et
la détermination (218) si l'évaluation du système moteur est altérée ou non.

10. Procédé selon la revendication 9, comprenant en outre :
l'affichage d'une instruction, dans lequel l'instruction ne constitue pas un traitement pour une évaluation du système moteur non altéré ; et
l'affichage d'une instruction, dans lequel l'instruction consiste à initier un traitement pour une évaluation d'un système moteur altéré.

11. Procédé selon la revendication 10, comprenant en outre :
le calcul du traitement basé sur l'évaluation du système moteur, dans lequel le traitement consiste à administrer au patient une quantité thérapeutiquement efficace de médicament.

12. Système d'évaluation des symptômes moteurs chez les patients atteints de la maladie de Parkinson comprenant :
un affichage (119) ;
un capteur ;
un processeur (111) ; et
une mémoire (121), dans lequel la mémoire est en communication avec le processeur stockant des instructions qui, lorsqu'elles sont exécutées par le processeur, amènent le système à :
obtenir, à partir du capteur, des données de mouvement du patient dans lequel les données de mouvement comprennent des données de mouvement passif et des données de performance active, dans lequel les données de mouvement passif du patient comprennent la puissance du geste, la puissance du pas, l'étendue de la marche, la fréquence du tremblement pathologique de la main, le temps du geste, la vitesse de rotation et la durée de l'étendue du geste ;
déterminer si les données relatives aux mouvements du patient sont altérées ou non ; et
générer, sur la base des données de mouvement du patient, une évaluation du système moteur, dans lequel l'évaluation du système moteur est altérée ou non.

13. Système selon la revendication 12, dans lequel l'évaluation du système moteur est fortement corrélée aux données de mesure de l'état de santé EQ-5D-5L, et dans lequel la puissance du geste est indicative des données de mobilité EQ-5D-5L ;
dans lequel la puissance du geste, la puissance du pas, l'étendue de la marche et la fréquence des tremblements pathologiques de la main sont indicatives des données d'autosoins EQ-5D-5L ;
dans lequel les données relatives à la puissance du geste, à la puissance du pas et au temps de geste sont indicatives des données relatives aux activités habituelles de l'EQ-5D-5L ;
dans lequel la puissance de geste et/ou la vitesse de rotation indiquées correspondent à des données douleur/inconfort de l'EQ-5D-5L ; et
dans lequel le temps de geste, la durée de l'étendue du geste et les données d'étendue de la marche sont indicatives des données d'anxiété/de dépression de l'EQ-5D-5L.

14. Système selon la revendication 12, dans lequel les instructions, lorsqu'elles sont lues par le processeur, amènent le système à comprendre en outre :
le calcul, sur la base des données de mouvement de population de patients dans lequel les données de mouvement comprennent des données de mouvement passif et/ou des données de performance active, d'une valeur seuil définissant l'évaluation du système moteur comme étant altérée ou non ;
le calcul d'un mouvement/score de performance du patient ;
la comparaison du mouvement/score de performance du patient à la valeur seuil ;
la détermination si l'évaluation du système moteur est altérée ou non ;
l'affichage d'une instruction, dans lequel l'instruction ne constitue pas un traitement pour une évaluation du système moteur non altéré ;
l'affichage d'une instruction, dans lequel l'instruction consiste à initier un traitement pour une évaluation d'un système moteur altéré ; et
le calcul du traitement basé sur l'évaluation du système moteur, dans lequel le traitement consiste à administrer au patient une quantité thérapeutiquement efficace de médicament pour le patient atteint de la maladie de Parkinson.
